# EUROPÄISCHE PATENTANMELDUNG

(11) **EP 4 154 797 A1**
(43) Veröffentlichungstag der Anmeldung: **29.03.2023**
(21) Anmeldenummer: 21198221.0
(22) Anmeldetag: 22.09.2021
(51) Int. Cl.: A61B 5/00, A61B 5/055, G01R 33/54, G01R 33/56, G06T 7/00

(54) **VERFAHREN ZUM BESTIMMEN EINER DIAGNOSTISCH RELEVANTEN SCHNITTEBENE**

(71) Anmelder: Siemens Healthcare GmbH, 91052 Erlangen (DE)
(72) Erfinder: Dr. Giese, Daniel, 91052 Erlangen (DE); Schmidt, Michaela, 91080 Uttenreuth (DE); Wetzl, Jens, 91080 Spardorf (DE)

(57) **Zusammenfassung**

Ein computerimplementiertes Verfahren zum Bestimmen einer Ausrichtung wenigstens einer diagnostisch relevanten Schnittebene für eine Herzbildgebung in einem dreidimensionalen Magnet-Resonanz-Tomographie Bilddatensatz umfasst einen Verfahrensschritt eines Bereitstellens (PROV-1) des dreidimensionalen Bilddatensatzes. Das Verfahren umfasst außerdem einen Verfahrensschritt eines Anwendens (APP) einer trainierten Funktion auf den dreidimensionalen Bilddatensatz. Dabei wird eine Position wenigstens einer Landmarke bestimmt wird. Das Verfahren umfasst außerdem einen Verfahrensschritt eines Bestimmens (DET) der Ausrichtung der wenigstens einen diagnostisch relevanten Schnittebene in Abhängigkeit der wenigstens einen Landmarke. Das Verfahren umfasst außerdem einen Verfahrensschritt eines Bereitstellens (PROV-2) der Ausrichtung der wenigstens einen diagnostisch relevanten Schnittebene.

## Beschreibung

Die vorliegende Erfindung betrifft ein computerimplementiertes Verfahren zum Bestimmen einer Ausrichtung wenigstens einer diagnostisch-relevanten Schnittebene für eine Herzbildgebung in einem dreidimensionalen Magnet-Resonanz-Tomographie Bilddatensatz und ein Bestimmungssystem, welches ausgebildet ist, das Verfahren auszuführen. Die Erfindung betrifft ferner ein Magnet-Resonanz-Tomographie System, ein Computerprogrammprodukt und ein computerlesbares Speichermedium. Die Erfindung betrifft ferner ein computerimplementiertes Verfahren zum Bereitstellen einer trainierten Funktion zum Bestimmen einer Position wenigstens einer Landmarke in einem dreidimensionalen Magnet-Resonanz-Tomographie Bilddatensatz.

In einer Magnet-Resonanz-Tomographie (Akronym: MRT) Bildgebung kann ein zweidimensionales Schnittbild einer Schnittebene eines Untersuchungsobjektes mit einem MRT System erfasst werden. Die Schnittebene kann eine beliebige Ausrichtung relativ zu dem Untersuchungsobjekt bzw. relativ zu dem MRT System aufweisen. Das Untersuchungsobjekt kann dabei ein Mensch, insbesondere ein Patient oder ein Tier sein. Typische Ausrichtungen einer Schnittebene sind coronal, sagittal und axial. Diese Ausrichtungen werden auch als "Standardausrichtungen" bezeichnet.

Zum Erfassen des Schnittbildes ist das Untersuchungsobjekt in einem Aufnahmebereich des MRT Systems positioniert. Innerhalb des Aufnahmebereiches liegt ein Magnetfeld an. Das Magnetfeld setzt sich aus einem homogenen Grundmagnetfeld und einem Magnetfeldgradienten zusammen. Die Protonen-Spins des Untersuchungsobjektes in dem Aufnahmebereich richten sich entlang dieses Magnetfeldes aus. Die Stärke des Magnetfeldes bestimmt dabei eine Larmor-Frequenz, mit welcher die Spins präzedieren. Durch Einstrahlen eines Anregungspulses, werden genau die Spins angeregt, deren Larmor-Frequenz der Frequenz des Anregungspulses entspricht. Die angeregten Spins sind relativ zu dem Magnetfeld gekippt und präzedieren in Phase. In Abhängigkeit des die angeregten Spins umgebenden Materials relaxieren die Spins wieder. Dabei richten sich die Spins wieder entlang des Magnetfeldes aus und dephasieren. Die Dauer, bis die Spins wieder ausgerichtet sind und die Dauer des Dephasierens hängen von dem die Spins umgebenden Material ab. Ein von den Spins ausgesendetes elektromagnetisches Signal beim Relaxieren kann erfasst werden. Aus diesem Signal kann das Schnittbild oder dreidimensionale MRT Bilddaten rekonstruiert werden.

Der Magnetfeldgradient dient zur Ortskodierung. Durch den Magnetfeldgradienten ist die Magnetfeldstärke an verschiedenen Orten des Untersuchungsobjektes innerhalb des Aufnahmebereiches unterschiedlich. Damit präzedieren auch die Spins mit einer unterschiedlichen Larmor-Frequenz in Abhängigkeit von ihrer Position in dem Magnetfeld bzw. in dem Untersuchungsobjekt. Durch eine geeignete Wahl der Frequenz des Anregungspulses können also nur bestimmte Spins angeregt werden. Auf diese Weise können in Abhängigkeit der Ausrichtung des Magnetfeldgradienten und der Frequenz des Anregungspulses Spins in einer beliebige Schnittebene angeregt werden. Die mit dem Anregungspuls angeregten Spins, das heißt die Spins in der Schnittebene, präzedieren alle mit annähernd derselben Frequenz und sind innerhalb eines annähernd konstanten Bereichs des Magnetfeldgradienten angeordnet. Das erfasste Signal beschreibt damit die Relaxation der Spins in der Schnittebene. Auf diese Weise kann das Schnittbild der Schnittebene rekonstruiert werden. Durch (paralleles) Erfassen von mehr als einem Schnittbild kann ein dreidimensionaler Bilddatensatz rekonstruiert werden.

Das Herz ist bei verschiedenen Untersuchungsobjekten verschieden ausgerichtet. Um eine Diagnose oder Untersuchung bezüglich des Herzens durchführen zu können, muss ein Schnittbild des Herzens eine bestimmte Sicht auf das Herz umfassen bzw. abbilden. Insbesondere muss das Schnittbild eine diagnostisch relevante Schnittebene des Herzens abbilden. Die Ausrichtung der diagnostisch relevante Schnittebene ist typischerweise durch eine Position wenigstens einer Landmarke in dem Herzen vorgegeben bzw. abhängig. Durch die individuelle Ausrichtung des Herzens ist es nicht möglich, das Schnittbild des Herzens mit einer der Standardausrichtungen (coronal, sagittal, axial) zu erfassen.

Es ist bekannt, dass eine Ausrichtung einer diagnostisch relevanten Schnittebene für ein Schnittbild iterativ von einem erfahrenen medizinischen Bedienpersonal bestimmt wird. Dafür wird zunächst eine erste Mehrzahl von zweidimensionalen Schnittbildern beispielsweise gemäß den Standardausrichtungen erfasst. Basierend auf dieser ersten Mehrzahl von Schnittbildern kann das medizinische Bedienpersonal eine Ausrichtung einer oder mehrerer weiterer Schnittebenen bestimmen. Von diesen Schnittebenen werden wiederum Schnittbilder erfasst, die wiederum zum Bestimmen einer Ausrichtung einer oder mehrerer weiterer Schnittebenen dienen, von welchen Schnittbilder erfasst werden. Insbesondere kann in jedem erfassten Schnittbild wenigstens eine Landmarke durch das medizinische Bedienpersonal bestimmt werden. Basierend auf dieser wenigstens einen Landmarke bzw. deren Position in dem Schnittbild kann die Ausrichtung der folgenden Schnittebenen bzw. der Schnittebenen der nächsten Iteration bestimmt werden kann. Auf diese Weise kann iterativ die Schnittebene bestimmt werden, die die wenigstens eine Landmarke umfasst, die wiederrum die Ausrichtung der diagnostisch relevanten Schnittebene vorgibt. Von dieser Schnittebene kann dann die diagnostisch relevante Schnittebene bzw. deren Ausrichtung abgeleitet werden. Eine mögliche Ausführung dieses iterativen Verfahrens ist beispielsweise von Maier et al. In "Kardiale Magnetresonanztomographie - Anatomie und Planung", Journal für Kardiologie - Austrian Journal of Cardiology 2003; 10: 3-7 beschrieben.

Blansit et al. beschreiben in "Deep Learning-based Prescription of Cardiac MRI Planes", Radiology: Artificial Intelligence 2019; 1(6):e180069 ein Verfahren zum Bestimmen der Landmarken in den zweidimensionalen Schnittbildern mittels einer trainierten Funktion. Auf diese Weise kann das oben beschriebene iterative Verfahren automatisiert werden.

Dennoch ist es sowohl bei dem manuellen iterativen Verfahren als auch bei dem automatisierten iterativen Verfahren notwendig, eine große Anzahl von Schnittbildern zu erfassen, die nicht diagnostisch relevant sind und lediglich dazu dienen eine Ausrichtung des Herzens in dem Untersuchungsobjekt zu bestimmen und daraus die diagnostisch relevante Schnittebene abzuleiten.

Das beschriebene iterative Verfahren zum Bestimmen der wenigstens einen diagnostisch relevanten Schnittebene ist sehr zeitaufwendig. Somit ist das iterative Verfahren außerdem sehr kostenintensiv. Das Untersuchungsobjekt muss zum Erfassen der einzelnen Schnittbilder still bzw. möglichst unbeweglich in dem typischerweise engen Aufnahmebereich des MRT Systems positioniert bleiben. Zudem kann es notwendig sein, dass das Untersuchungsobjekt für jedes Schnittbild seinen Atem anhalten muss. Dies führt zu einem hohen Diskomfort für das Untersuchungsobjekt bereits im Vorfeld der eigentlichen Untersuchung, bzw. dem Erfassen des Schnittbildes der diagnostisch relevanten Schnittebene.

Es ist daher die Aufgabe der vorliegenden Erfindung, ein Verfahren bereitzustellen, das ein schnelles und automatisiertes Bestimmen einer diagnostisch relevanten Schnittebene ermöglicht.

Die Aufgabe wird gelöst durch ein computerimplementiertes Verfahren zum Bestimmen einer Ausrichtung wenigstens einer diagnostisch-relevanten Schnittebene für eine Herzbildgebung in einem dreidimensionalen Magnet-Resonanz-Tomographie Bilddatensatz, durch ein computerimplementiertes Verfahren zum Bereitstellen einer trainierten Funktion zum Bestimmen einer Position wenigstens einer Landmarke in einem dreidimensionalen Magnet-Resonanz-Tomographie Bilddatensatz, durch ein Bestimmungssystem, durch ein Magnet-Resonanz-Tomographie System, durch ein Computerprogrammprodukt und durch ein computerlesbares Speichermedium gemäß den unabhängigen Ansprüchen. Vorteilhafte Weiterbildungen sind in den abhängigen Ansprüchen und in der folgenden Beschreibung aufgeführt.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Weiterhin wird die erfindungsgemäße Lösung der Aufgabe sowohl in Bezug auf Verfahren und Vorrichtungen zum Bestimmen einer Ausrichtung wenigstens einer diagnostisch-relevanten Schnittebene für eine Herzbildgebung in einem dreidimensionalen Magnet-Resonanz-Tomographie Bilddatensatz als auch in Bezug auf Verfahren und Vorrichtungen zum Bereitstellen einer trainierten Funktion beschrieben. Hierbei können Merkmale und alternative Ausführungsformen von Datenstrukturen und/oder Funktionen bei Verfahren und Vorrichtungen zur Bestimmung auf analoge Datenstrukturen und/oder Funktionen bei Verfahren und Vorrichtungen zum Anpassen/Optimieren/Trainieren übertragen werden. Analoge Datenstrukturen können hierbei insbesondere durch die Verwendung der Vorsilbe "Trainings" gekennzeichnet sein. Weiterhin können die in Verfahren und Vorrichtungen zum Bestimmen einer Ausrichtung wenigstens einer diagnostisch-relevanten Schnittebene für eine Herzbildgebung in einem dreidimensionalen Magnet-Resonanz-Tomographie Bilddatensatz verwendeten trainierten Funktionen insbesondere durch Verfahren zum Bereitstellen der trainierten Funktion trainiert bzw. angepasst worden und/oder bereitgestellt worden sein.

Die Erfindung betrifft ein computerimplementiertes Verfahren zum Bestimmen einer Ausrichtung wenigstens einer diagnostisch relevanten Schnittebene für eine Herzbildgebung in einem dreidimensionalen Magnet-Resonanz-Tomographie Bilddatensatz. Das Verfahren umfasst einen Verfahrensschritt eines Bereitstellens des dreidimensionalen Bilddatensatzes. Das Verfahren umfasst außerdem einen Verfahrensschritt eines Anwendens einer trainierten Funktion auf den dreidimensionalen Bilddatensatz. Dabei wird eine Position wenigstens einer Landmarke bestimmt. Das Verfahren umfasst einen Verfahrensschritt eines Bestimmens der Ausrichtung der wenigstens einen diagnostisch relevanten Schnittebene in Abhängigkeit der wenigstens einen Landmarke. Das Verfahren umfasst einen Verfahrensschritt eines Bereitstellens der Ausrichtung der wenigstens einen diagnostisch relevanten Schnittebene.

Der dreidimensionale Magnet-Resonanz-Tomographie (Akronym: MRT) Bilddatensatz bildet wenigstens einen Teil eines Untersuchungsobjektes ab. Das Untersuchungsobjekt ist dabei ein Mensch, insbesondere ein Patient, oder ein Tier. Der abgebildete Teil des Untersuchungsobjektes umfasst wenigstens einen Teil des Herzens des Untersuchungsobjektes. Der dreidimensionale MRT Bilddatensatz (im Folgenden auch als dreidimensionaler Bilddatensatz bezeichnet) bzw. die dreidimensionale MRT Aufnahme umfasst eine dreidimensionale Abbildung des Herzens bzw. wenigstens des Teils des Herzens.

Der dreidimensionale Bilddatensatz umfasst eine Mehrzahl von Voxeln, die in einer dreidimensionalen Voxelmatrix angeordnet sind. Jedem Voxel kann dabei eine Position in dem Untersuchungsobjekt zugeordnet werden. Jeder Voxel umfasst einen Voxelwert, der einen Intensitätswert darstellt, der von einem von der entsprechenden Position des Untersuchungsobjektes ausgesendeten Signal abgeleitet ist. Der Intensitätswert kann einen Grauwert oder einen Farbwert des Voxels in einer Darstellung des dreidimensionalen Bilddatensatzes vorgeben.

In Ausführungen der Erfindung kann der dreidimensionale Bilddatensatz auch eine zweidimensionale Multischicht Aufnahme bzw. eine Multischicht 2D Aufnahme sein. Dabei kann eine Mehrzahl von zweidimensionalen Schichtbildern bzw. Schnittbildern des Herzens bzw. des Teils des Herzens in verschiedenen Ebenen räumlich sequenziell erfasst worden sein. Die zweidimensionalen Schichtbilder sind dabei parallel zueinander ausgerichtet. Die zweidimensionalen Schichtbilder der Multischicht Aufnahme können "gestapelt" also hintereinander angeordnet werden. Die "gestapelten" zweidimensionalen Schichtbilder bilden gemeinsam eine "pseudo" dreidimensionale Abbildung des Herzens bzw. des Teils des Herzens. Jedes Schichtbild umfasst dabei eine Mehrzahl von Pixeln, die in einer zweidimensionale Pixelmatrix angeordnet sind. Jedem Pixel ist dabei ein Pixelwert zugeordnet, der analog zu dem oben beschriebenen Voxelwert ausgebildet ist. Teile der folgenden Beschreibung, die sich auf die Voxel bzw. Voxelwerte des dreidimensionalen Bilddatensatzes beziehen können analog auf die Pixel bzw. Pixelwerte der zweidimensionalen Multischicht Aufnahme übertragen werden.

Der dreidimensionale Bilddatensatz kann eine Übersichtsaufnahme des Teils des Untersuchungsobjektes sein. Mit anderen Worten kann der dreidimensionale Bilddatensatz mit einem schnellen Aufnahmeprotokoll erfasst worden sein. Insbesondere kann das Aufnahmeprotokoll zum Erfassen der Übersichtsaufnahme bezüglich einer Aufnahmedauer optimiert sein. Dafür kann eine Bildqualität, beispielsweise eine räumliche Auflösung und/oder ein Signal-zu-Rausch Verhältnis, zu Gunsten einer kürzeren Aufnahmezeit reduziert sein. Insbesondere kann eine räumliche Auflösung des dreidimensionalen Bilddatensatzes für eine diagnostische Auswertung zu gering sein. Insbesondere ist die Auflösung ausreichend, um die wenigstens eine Landmarke bzw. deren Position in dem dreidimensionalen Bilddatensatz bestimmen zu können.

Die diagnostisch relevante Schnittebene bzw. Schichtebene ist dabei eine zweidimensionale Schnittebene durch den dreidimensionalen Bilddatensatz. Die Ausrichtung der diagnostisch relevanten Schnittebene kann dabei relativ zu dem dreidimensionalen Bilddatensatz bestimmt werden. Insbesondere kann dann die Ausrichtung relativ zu einem MRT System, mit dem der dreidimensionale Bilddatensatz erfasst wurde, bestimmt werden. Mit anderen Worten kann die Ausrichtung der diagnostisch relevanten Schnittebene auf ein Koordinatensystem des MRT Systems bezogen sein. Die Ausrichtung beschreibt dabei die räumliche Lage bzw. die räumliche Ausrichtung der diagnostisch relevanten Schnittebene. Mit anderen Worten kann die Ausrichtung eine Angulation der diagnostisch relevanten Schnittebene beschreiben. Insbesondere kann die räumliche Lage beispielsweise durch eine Richtung eines Normalenvektors auf die diagnostisch relevante Schnittebene beschrieben werten. In Ausführungen der Erfindung kann die Ausrichtung außerdem eine Position der diagnostisch relevanten Schnittebene beschreiben. Die Position gibt dabei an, wo die diagnostisch relevante Schnittebene das Untersuchungsobjekt schneiden soll, bzw. welche Punkte in der diagnostisch relevanten Schnittebene liegen sollten. Mit anderen Worten umfasst die Ausrichtung eine Information über die räumliche Ausrichtung bzw. räumliche Lage der diagnostisch relevanten Schnittebene und in Ausführungen der Erfindung eine Information über die Position der diagnostisch relevanten Schnittebene.

"Diagnostisch relevant" bedeutet, dass basierend auf einem zweidimensionalen Schnittbild bzw. Schichtbild der diagnostisch relevanten Schnittebene eine Diagnose für das Untersuchungsobjekt erstellt werden kann bzw. eine Untersuchung durchgeführt werden kann. Das Schnittbild bildet dabei einen Schnitt durch das Untersuchungsobjekt in der diagnostisch relevanten Schnittebene ab. Insbesondere kann das Schnittbild einen vordefinierten bzw. standardisierten Querschnitt des Herzens des Untersuchungsobjektes abbilden. Mit anderen Worten ist die Ausrichtung der diagnostisch relevanten Schnittebene derart gewählt, dass das entsprechende Schnittbild den vordefinierten bzw. standardisierten Querschnitt des Herzens abbildet. Die Diagnose kann beispielsweise eine Diagnose einer Myokarditis oder eine Perikardergusses oder eines Herzinfarktes oder eines Tumors oder einer Erkrankung einer Herzklappe etc. sein.

In dem Verfahrensschritt des Bereitstellens des dreidimensionalen Bilddatensatzes kann der dreidimensionale Bilddatensatz insbesondere empfangen oder erfasst oder abgerufen werden.

Der dreidimensionale Bilddatensatz kann insbesondere mittels einer Schnittstelle empfangen oder abgerufen werden. Der dreidimensionale Bilddatensatz kann beispielsweise von einer Datenbank abgerufen oder empfangen werden. Die Datenbank kann dabei eine Bilddatenbank einer medizinischen Einrichtung beispielsweise eines Krankenhauses sein. Die Datenbank kann insbesondere ein Bildarchivierungs- und Kommunikationssystem (engl. Picture Archiving and Communication System, Akronym: PACS) sein. Die Datenbank kann auf einem Server gespeichert sein. Der Server kann ein lokaler Server oder ein Cloud-Server sein. Der dreidimensionale Bilddatensatz kann somit in dem Verfahrensschritt des Bereitstellens des dreidimensionalen Bilddatensatzes von dem Server abgerufen bzw. empfangen bzw. bereitgestellt werden.

Alternativ kann der dreidimensionale Bilddatensatz in dem Verfahrensschritt des Bereitstellens des dreidimensionalen Bilddatensatzes erfasst werden. Mit anderen Worten kann in dem Verfahrensschritt ein Signal mit dem MRT System erfasst werden, aus welchem der dreidimensionale Bilddatensatz rekonstruiert werden kann. Das Signal kann dabei wie oben beschrieben auf einer Relaxation von Spins in dem abgebildeten Teil des Untersuchungsobjektes basieren.

In dem Verfahrensschritt des Anwendens der trainierten Funktion wird die trainierte Funktion auf den dreidimensionalen Bilddatensatz angewendet, um die Position der wenigstens einen Landmarke zu bestimmen.

Insbesondere kann dabei jeweils eine Position für mehr als eine Landmarke bestimmt werden. Die Landmarke beschreibt dabei eine Anatomie des Herzens. Die Landmarke kann beispielsweise eine Herzklappe oder ein venöser bzw. arterieller Eingang oder Ausgang des Herzens oder der Apex etc. sein. Die Position der Landmarke kann dabei durch die Position des wenigstens einen Voxels, der die Landmarke in dem wenigstens einen dreidimensionalen Bilddatensatz abbildet, in der Voxelmatrix beschrieben werden. Insbesondere kann aus der Position des Voxels auf die Position der Landmarke in dem Untersuchungsobjekt geschlossen werden.

Im Folgenden umfasst die Bezeichnung "die wenigstens eine Landmarke" immer auch die Position der wenigstens einen Landmarke.

In dem Verfahrensschritt des Anwendens der trainierten Funktion wird mittels der trainierten Funktion basierend auf den dreidimensionalen Bilddaten die Position der wenigstens einen Landmarke erzeugt.

Im Allgemeinen ahmt eine trainierte Funktion kognitive Funktionen nach, die Menschen mit menschlichem Denken verbinden. Insbesondere durch auf Trainingsdaten basierendem Training kann sich die trainierte Funktion an neue Umstände anpassen sowie Muster erkennen und extrapolieren.

Im Allgemeinen können Parameter einer trainierten Funktion mittels Trainings angepasst werden. Insbesondere kann dafür ein beaufsichtigtes (supervised) Training, ein halbüberwachtes (semi-supervised) Training, ein unbeaufsichtigtes (unsupervised) Training, ein verstärkendes Lernen (reinforcement learning) und/oder ein aktives Lernen (active learning) verwendet werden. Darüber hinaus kann Repräsentationslernen (ein alternativer Begriff ist "Merkmalslernen") (representation learning bzw. feature learning) verwendet werden. Insbesondere können die Parameter der trainierten Funktionen durch mehrere Trainingsschritte iterativ angepasst werden.

Insbesondere kann eine trainierte Funktion ein neuronales Netzwerk, eine Unterstützungsvektormaschine (support vector machine), einen Zufallsbaum bzw. einen Entscheidungsbaum (decision tree) und/oder ein Bayes'sches Netzwerk umfassen, und/oder die trainierte Funktion kann auf k-Mittel-Clustering (k-means clustering), Q-Learning, genetischen Algorithmen und/oder Assoziationsregeln basieren. Insbesondere kann eine trainierte Funktion eine Kombination aus mehreren unkorrelierten Entscheidungsbäumen bzw. ein Ensemble aus Entscheidungsbäumen (random forest) umfassen. Insbesondere kann die trainierte Funktion mittels XGBoosting (eXtreme Gradient Boosting) bestimmt werden. Insbesondere kann ein neuronales Netzwerk ein tiefes neuronales Netzwerk (deep neural network), ein Faltungs-neuronales Netzwerk (convolutional neural network) oder ein Faltungs-tiefes neuronales Netzwerk (convolutional deep neural network) sein. Darüber hinaus kann ein neuronales Netzwerk ein kontradiktorisches Netzwerk (adversarial network), ein tiefes kontradiktorisches Netzwerk (deep adversarial network) und/oder ein generatives kontradiktorisches Netzwerk (generative adversarial network) sein. Insbesondere kann ein neuronales Netzwerk ein rekurrentes neuronales Netzwerk (recurrent neural network) sein. Insbesondere kann ein rekurrentes neuronales Netzwerk ein Netzwerk mit langem Kurzzeitgedächtnis (long-short-term-memory, LSTM), insbesondere eine Gated Recurrent Unit (GRU), sein. Insbesondere kann eine trainierte Funktion eine Kombination der beschriebenen Ansätze umfassen. Insbesondere werden die hier beschriebenen Ansätze für eine trainierte Funktion Netzwerkarchitektur der trainierten Funktion genannt.

In dem Verfahrensschritt des Bestimmens der Ausrichtung der wenigstens einen diagnostisch relevanten Schnittebene, wird in Abhängigkeit der wenigstens einen Landmarke die Ausrichtung der diagnostisch relevanten Schnittebene bestimmt. Mit anderen Worten wird die Ausrichtung der wenigstens einen diagnostisch relevanten Schnittebene in Abhängigkeit der Position der wenigstens einen Landmarke bestimmt.

Insbesondere kann in dem Verfahrensschritt die Ausrichtung von mehr als einer diagnostisch relevanten Schnittebene bestimmt werden.

Insbesondere kann die Ausrichtung der wenigstens eine diagnostisch relevante Schnittebene in Abhängigkeit von mehr als einer Landmarke bzw. deren Positionen bestimmt werden.

Beispielsweise kann die Ausrichtung der diagnostisch relevanten Schnittebene durch die Positionen von drei oder mehr Landmarken vorgegeben werden. Dabei kann die diagnostisch relevante Schnittebene derart ausgerichtet sein, dass die drei oder mehr Landmarken in der Schnittebene liegen.

Alternativ kann die Ausrichtung der diagnostisch relevanten Schnitteben von der Position von einer oder zwei Landmarken abhängen. Dabei kann die diagnostisch relevante Schnittebene derart ausgerichtet sein, dass die eine oder beide Landmarken in der Schnittebene liegen. Dann kann die Ausrichtung außerdem von einem Vorwissen beispielsweise einer Standardausrichtung (coronal, sagittal oder axial) abhängen.

Alternativ kann die Ausrichtung der diagnostisch relevanten Schnittebene durch eine Ausrichtung relativ zu der wenigstens einen Landmarke vorgegeben sein. Beispielsweise kann die diagnostisch relevante Schnittebene zu einer von drei Landmarken aufgespannten Ebene senkrecht sein.

Wie oben beschrieben gibt die Ausrichtung die Ausrichtung der diagnostisch relevanten Schnittebene relativ zu dem dreidimensionalen Bilddatensatz bzw. zu dem MRT System an.

In dem Verfahrensschritt des Bereitstellens der Ausrichtung der wenigstens einen diagnostisch relevanten Schnittebene, wird die Ausrichtung insbesondere mittels einer Schnittstelle bereitgestellt.

Die Ausrichtung kann dabei beispielsweise als Normalenvektor auf die wenigstens eine diagnostisch relevante Schnittebene in einem Koordinatensystem des MRT Systems angegeben werden. Alternativ oder zusätzlich kann die Ausrichtung derart angegeben werden, dass eine Gradientensteuereinheit zu einem Erzeugen eines Magnetfeldgradienten angesteuert werden kann. Dabei kann die Gradientensteuereinheit wenigstens eine Gradientenspule derart ansteuern, dass ein Magnetfeldgradient erzeugt wird, der senkrecht auf die wenigstens einen diagnostisch relevante Schnittebene steht. Dabei kann der Magnetfeldgradient derart ausgebildet sein, dass in der Ebene der wenigstens einen diagnostisch relevanten Schnittebene ein Magnetfeld mit einer vordefinierten. konstanten Magnetfeldstärke anliegt.

Die Erfinder haben erkannt, dass in dem dreidimensionalen Bilddatensatz mit einer trainierten Funktion alle relevanten Landmarken auf einmal bestimmt werden können. Damit ist es ausreichend, eine Aufnahme des Untersuchungsobjektes zum Planen der wenigstens einen diagnostisch relevanten Schnittebene bzw. zum Bestimmen deren Ausrichtung, im Vorfeld zu erfassen. Somit ist kein iteratives Verfahren zum Bestimmen der Ausrichtung der wenigstens einen diagnostisch relevanten Schnittebene mehr notwendig. Auf diese Weise kann der Komfort des Untersuchungsobjektes erhöht werden, da das Verfahren zum Bestimmen der diagnostisch relevanten Schnittebene beschleunigt werden kann und lediglich eine Aufnahme zur Planung der diagnostisch relevanten Schnittebene notwendig ist. Dies führt außerdem zu einer Kostenersparnis. Insbesondere kann durch das Bestimmen aller Landmarken in einem dreidimensionalen Bilddatensatz ein räumlicher Zusammenhang zwischen verschiedenen Landmarken bestimmt werden, da die Positionen aller Landmarken in einem einzigen dreidimensionalen Bilddatensatz bestimmt werden. Dies konnte in den bisherigen Verfahren nicht sichergestellt werden, da dort die Positionen in verschiedenen, räumlich unzusammenhängenden zweidimensionalen Bilddatensätzen bestimmt wurden. Eine Fehlerpropagation durch ein iteratives Verfahren kann durch das erfindungsgemäße Verfahren vermieden werden.

Nach einem Aspekt des Verfahrens bildet der dreidimensionale Bilddatensatz wenigstens einen Teil eines Herzens ab. Dabei ist der dreidimensionale Bilddatensatz ein Übersichtsscan des Herzens.

Der dreidimensionale Bilddatensatz bildet dabei wenigstens einen Teil des Herzens des Untersuchungsobjektes ab. Insbesondere kann der dreidimensionale Bilddatensatz das gesamte Herz des Untersuchungsobjektes abbilden.

Der Übersichtsscan ist dabei wie oben beschrieben ausgebildet. Insbesondere wird bei dem Übersichtsscan eine geringere Bildqualität, insbesondere ein höheres Rauschen und/oder eine reduzierte räumliche Auflösung, für ein schnelleres Erfassen des dreidimensionalen Bilddatensatzes bzw. eine kürzere Aufnahmedauer in Kauf genommen.

Insbesondere kann der Übersichtsscan auch eine zweidimensionale Multischicht Aufnahme sein, die wie oben beschrieben ausgebildet sein kann.

Die Erfinder haben erkannt, dass ein Übersichtsscan des Herzens bzw. des Teils des Herzens ausreichend ist, um die Position der wenigstens einen Landmarke zu bestimmen. Insbesondere ist eine geringere Bildqualität, insbesondere ein höheres Rauschen und/oder eine geringere räumliche Auflösung, in dem dreidimensionalen Bilddatensatz tolerierbar, da der dreidimensionale Bilddatensatz nicht zum Erstellen einer Diagnose oder einer Untersuchung erfasst wird. Auf diese Weise kann eine Zeit zum Erfassen des dreidimensionalen Bilddatensatzes minimiert werden und das Verfahren zum Bestimmen der Ausrichtung der wenigstens einen diagnostisch relevanten Schnittebene beschleunigt werden.

Nach einem weiteren Aspekt der Erfindung ist die wenigstens eine Landmarke eine der folgenden Landmarken: Apex, Mitralklappe, Aortenklappe, Pulmonalklappe, Trikuspidalklappe.

Insbesondere kann die Position der Landmarke einen markanten Punkt des Apex, der Mitralklappe, der Aortenklappe, der Pulmonalklappe oder der Trikuspidalklappe betreffen bzw. beschreiben bzw. markieren.

In Ausführungen der Erfindung kann die wenigstens eine Landmarke eine der folgenden Landmarken sein:
- Links Ventrikulärer Apex (engl.: left ventricular apex, Akronym: LV apex)
- Rechts Ventrikulärer Apex (engl.: right ventricular apex, Akronym: RV apex)
- Rechtes Ventrikel Ausdehnung (engl.: right ventricle extent, Akronym: RV extent)
- Mitralklappe, Zentrum (engl.: mitral valve, midpoint, Akronym: MV midpoint)
- Aortenklappe, Zentrum (engl.: aortic valve, midpoint, Akronym: AV midpoint)
- Trikuspidalklappe, Zentrum (engl.: tricuspid valve, midpoint, Akronym: TV midpoint)
- Pulmonalklappe, Zentrum (engl.: pulmonary valve, midpoint, Akronym: PA center point)
- Aortenbogen (engl.: aortic arch)
- Absteigende Aorta (engl.: descending aorta)
- Vier Punkte auf der Gefäßwand der Aortenwurzel (anterior, posterior, links, rechts) (engl.: aortic bulb)
- Vier Punkte auf der Gefäßwand der Pulmonalarterie (links, rechts, superior, inferior) an einer geeigneten Stelle für eine Flussmessung der Pulmonalarterie (engl.: pulmonary artery flow plane)
- Vier Punkte auf dem Annulus der Pulmonalklappe (links, rechts, superior, inferior) (engl.: pulmonary valve insertion)
- Vier Punkte auf dem Annulus der Mitralklappe (septal, lateral, superior, inferior) (engl.: mitral valve insertion)
- Vier Punkte auf dem Annulus der Trikuspidalklappe (septal, lateral, superior, inferior) (engl.: tricuspid valve insertion)
- Insertionspunkte des rechten Ventrikels (anterior, posterior) am linken Ventrikel (engl.: right ventricle insertion points)
- Mittellinie der absteigenden Aorta (engl.: descending aorta centerline)
- Mittellinie der aufsteigenden Aorta (engl.: ascending aorta centerline).

Die Erfinder haben erkannt, dass wenigstens eine vordefinierte bzw. eine standardisierte Landmarke bestimmt werden kann. Auf diese Weise kann aus einer bekannten räumlichen Relation die Ausrichtung der wenigstens einen diagnostisch relevanten Schnittebene von der Position der wenigstens einen Landmarke abgeleitet werden. Dadurch kann eine Vergleichbarkeit zwischen verschiedenen Untersuchungsobjekten und/oder zwischen Schnittbildern von einem Untersuchungsobjekt, die zu unterschiedlichen Zeitpunkten erfasst wurden, sichergestellt werden. Wenn die Ausrichtung der wenigstens einen diagnostisch relevanten Schnittebene auf wenigstens einer vordefinierten bzw. standardisierten Landmarke bzw. deren Position basiert, kann die Ausrichtung nach einem Standardverfahren bestimmt werden. Insbesondere kann dann die Ausrichtung einer standardisierten diagnostisch relevanten Schnittebene bestimmt werden.

Nach einem weiteren Aspekt der Erfindung ist die wenigstens eine diagnostisch relevante Schnittebene eine der folgenden Schnittebenen: Vier-Kammern-Ebene, Drei-Kammern-Ebene, Zwei-Kammern-Ebene, Vertikale-Lange-Achse, Horizontale-Lange-Achse, Kurze-Achse.

Die Lage der genannten Ebenen zueinander ist insbesondere in Maier et al., "Kardiale Magnetresonanztomographie - Anatomie und Planung", Journal für Kardiologie - Austrian Journal of Cardiology 2003; 10: 3-7 beschrieben.

Die wenigstens eine diagnostisch relevante Schnittebene kann in Ausführungen der Erfindung eine der folgenden Schnittebenen sein:
- Vertikale-Lange-Achse (engl.: vertical long axis, Akronym: VLA)
- Horizontale-Lange-Achse (engl.: horizontal long axis, Akronym: HLA)
- Drei-Kammern-Ebene (engl.: three chamber view, Akronym: 3CH)
- Vier-Kammern-Ebene (engl.: four chamber view, Akronym: 4CH)
- Kurze Achse (engl.: short axis, Akronym: SAX)
- Rechtes Ventrikel Zwei-Kammern-Ebene (engl.: right ventricle two chamber view, Akronym: RV-2CH)
- Rechtes Ventrikel Drei-Kammern-Ebene (engl.: right ventricle three chamber view, Akronym: RV-3CH)
- Aortenfluss-Ebene (engl.: Aortic Flow Plane)
- Ebene des linksvetrikulären Ausflusstrakts (engl.: left ventricular outflow tract plane, Akronym: LVOT)
- Ebene der Mitralklappe (engl.: mitral valve plane, Akronym: MV Plane).

Dabei kann die Ausrichtung der Schnittebenen von den im Folgenden hinter den Schnittebenen in Klammern angegebenen Landmarken bzw. deren Positionen abgeleitet werden bzw. abhängen:
- VLA (LV apex, MV midpoint + Vorwissen: Parasagittal)
- HLA (LV apex, MV midpoint + Vorwissen: orthogonal zu VLA)
- 3CH (LV apex, MV midpoint, AV midpoint)
- 4CH (LV apex, MV midpoint, RV extent)
- SAX (Mittelpunkt von LV apex und MV midpoint, RV extent + Vorwissen: orthogonal zu VLA und HLA)
- RV-2CH (RV apex, TV midpoint + Vorwissen: Parasagittal)
- RV-3CH (RV apex, TV midpoint, PA center point)
- Aortic Flow Plane (Aortic bulb (anterior), Aortic bulb (posterior), Aortic bulb (left), Aortic bulb (right))
- LVOT (Aortic bulb (anterior), Aortic bulb (posterior) + Vorwissen: Schicht im Mittelpunkt der beiden Landmarken, orthogonal zum Verbindungsvektor der Landmarken)
- MV Plane (MV insertion (septal), MV insertion (lateral), MV insertion (superior), MV insertion (inferior)).

Die Erfinder haben erkannt, dass die wenigstens eine diagnostisch relevante Schnittebene eine vordefinierte bzw. standardisierte Schnittebene sein kann. Die Erfinder haben erkannt, dass auf diese Weise eine Diagnose und/oder eine Untersuchung standardisiert werden kann. Insbesondere kann das medizinische Bedienpersonal, welches die Diagnose basierend auf dem Schnittbild der diagnostisch relevanten Schnittebene erstellt oder die Untersuchung basierend auf dem Schnittbild der diagnostisch relevanten Schnittebene durchführt, mit der Sicht in der diagnostisch relevanten Schnittebene vertraut sein. Auf diese Weise kann eine Diagnostik vereinheitlicht und beschleunigt werden.

Nach einem weiteren Aspekt der Erfindung wird in dem Verfahrensschritt des Anwendens der trainierten Funktion die Position der wenigstens einen Landmarke in Form einer Wahrscheinlichkeitsverteilung für die Position der wenigstens einen Landmarke in dem dreidimensionalen Bilddatensatz bestimmt.

Die Wahrscheinlichkeitsverteilung kann auch als "Heatmap" bezeichnet werden.

Insbesondere kann jedem Voxel des dreidimensionalen Bilddatensatzes eine Wahrscheinlichkeit zugeordnet werden, die angibt, wie wahrscheinlich die wenigstens eine Landmarke in dem entsprechenden Voxel abgebildet ist. Die Wahrscheinlichkeit kann in Ausführungen der Erfindung eine Zahl zwischen 0 und 1 sein. Dabei kann eine Wahrscheinlichkeit von "0" bedeuten, dass die wenigstens eine Landmarke in dem entsprechenden Voxel nicht abgebildet ist und "1" kann bedeuten, dass die wenigstens eine Landmarke in dem Voxel sicher abgebildet ist.

Insbesondere kann die Wahrscheinlichkeitsverteilung analog zu dem dreidimensionalen Bilddatensatz eine dreidimensionale Voxelmatrix umfassen. Dabei entspricht jeder Voxel dieser Voxelmatrix einem Voxel des dreidimensionalen Bilddatensatzes. Die Voxel beider Voxelmatrizen sind identisch angeordnet. Jeder Voxel der Wahrscheinlichkeitsverteilung umfasst die dem Voxel entsprechende, oben beschriebene Wahrscheinlichkeit als Voxelwert.

Wenn die Position von mehr als einer Landmarke bestimmt wird, kann für jede Landmarke eine eigene Wahrscheinlichkeitsverteilung bestimmt werden.

Alternativ kann die Wahrscheinlichkeitsverteilung bezüglich mehr als einer Landmarke in einer kombinierten Wahrscheinlichkeitsverteilung dargestellt bzw. beschrieben werden.

Die Erfinder haben erkannt, dass durch die Wahrscheinlichkeitsverteilung Unsicherheiten beim Bestimmen der Position der wenigstens einen Landmarke berücksichtigt werden können. Außerdem kann die Ausrichtung der wenigstens einen diagnostisch relevanten Schnittebene in Abhängigkeit von der Wahrscheinlichkeitsverteilung in einem gewissen Umfang variabel sein. Insbesondere kann die Ausrichtung der diagnostisch relevanten Schnittebene derart variiert werden, dass im Schnitt die Wahrscheinlichkeit der Voxel, die in der Schnittebene liegen, aller Landmarken von denen die Ausrichtung abhängt, maximal oder minimal ist.

Nach einem weiteren Aspekt der Erfindung umfasst der Verfahrensschritt des Bereitstellens der Ausrichtung der wenigstens einen diagnostisch relevanten Schnittebene einen Verfahrensschritt eines Bereitstellens wenigstens eines ersten Aufnahmeparameterwertes zum Steuern eines medizintechnischen Magnet-Resonanz-Tomographie Systems zum Erfassen eines zweitdimensionalen Schnittbildes von der wenigstens einen diagnostisch relevanten Schnittebene.

Insbesondere umfasst das MRT System wenigstens eine Gradientenspule, die mit einer von dem MRT System umfassten Gradientensteuereinheit ansteuerbar ist. In Ausführungen umfasst das MRT System mehr als eine Gradientenspule. Insbesondere sind die eine oder mehreren Gradientenspulen von einer Gradientenspuleneinheit umfasst.

Die wenigstens eine Gradientenspule kann mit der Gradientensteuereinheit derart angesteuert werden, dass die wenigstens eine Gradientenspule einen Magnetfeldgradienten erzeugt. Der Magnetfeldgradient wird einem Grundmagnetfeld in dem Aufnahmebereich des MRT Systems überlagert. Das Magnetfeld innerhalb des Aufnahmebereiches setzt sich somit aus dem homogenen Grundmagnetfeld und dem Magnetfeldgradienten zusammen.

Der erste Aufnahmeparameterwert kann insbesondere der Gradientensteuereinheit bereitgestellt werden. Die Gradientensteuereinheit kann basierend auf dem ersten Aufnahmeparameterwert die wenigstens eine Gradientenspule ansteuern. Insbesondere kann mit dem ersten Aufnahmeparameterwert eine Stärke und/oder eine Ausrichtung des Magnetfeldgradienten vorgegeben werden.

Vorteilhafterweise kann der Magnetfeldgradient bzw. die Ausrichtung des Magnetfeldgradienten senkrecht zu der wenigstens einen diagnostisch relevanten Schnittebene sein. Insbesondere ist der Magnetfeldgradient in der wenigstens einen diagnostisch relevanten Schnittebene dann konstant. Insbesondere ist eine Stärke des Magnetfeldes in der diagnostisch relevanten Schnittebene derart ausgebildet, dass die Spins in der diagnostisch relevanten Schnittebene mit einer Larmor-Frequenz präzedieren, die der Frequenz des Anregungspulses entspricht. Insbesondere kann, wenn das MRT System mehr als eine Gradientenspule umfasst, der erste Aufnahmeparameterwert angeben, welche Stromstärke an welcher Gradientenspule angelegt werden soll, damit der Magnetfeldgradient wie beschrieben ausgebildet ist.

Alternativ oder zusätzlich kann der erste Aufnahmeparameterwert eine "Steilheit" des Magnetfeldgradienten angeben, der mit der wenigstens einen Gradientenspule erzeugt werden soll. Auf diese Weise kann eine Schichtdicke einer in dem Schnittbild abgebildeten Schicht des Untersuchungsobjektes vorgegeben werden. Das Schnittbild bildet eine zweidimensionale Projektion der Schicht entlang ihrer Dicke ab. Je "steiler" der Magnetfeldgradient, desto dünner ist die projizierte Schicht. Je "flacher" der Magnetfeldgradient, desto dicker ist die projizierte Schicht.

Alternativ oder zusätzlich kann der erste Aufnahmeparameterwert eine Frequenz des Anregungspulses angeben. Dabei kann die Frequenz derart von der Stärke des Magnetfeldes bzw. des Magnetfeldgradienten abhängen, dass der Anregungspuls genau die Spins in der diagnostisch relevanten Schnittebene anregt. Insbesondere hängt die Frequenz des Anregungspulses somit auch von der Position der diagnostisch relevanten Schnittebene in dem Magnetfeld ab.

Das zweitdimensionale Schnittbild bildet einen Schnitt durch das Untersuchungsobjekt in der wenigstens einen diagnostisch relevanten Schnittebene ab. Das zweidimensionale Schnittbild kann mit dem MRT System erfasst werden.

Die Erfinder haben erkannt, mit einem ersten Aufnahmeparameterwert das MRT System derart angesteuert werden kann, dass ein zweidimensionales Schnittbild der diagnostisch relevanten Schnittebene erfasst werden kann. Insbesondere kann die Ausrichtung der diagnostisch relevanten Schnittebene den ersten Aufnahmeparameterwert umfassen. Mit anderen Worten kann die Ausrichtung der diagnostisch relevanten Schnittebene derart bereitgestellt werden, dass direkt das MRT System angesteuert werden kann.

Nach einem weiteren Aspekt der Erfindung wird der wenigstens eine erste Aufnahmeparameterwert von der Ausrichtung der wenigstens einen diagnostisch relevanten Schnittebene abgeleitet.

Insbesondere ist der wenigstens eine erste Aufnahmeparameterwert derart ausgebildet, dass das zweidimensionale Schnittbild in Abhängigkeit des ersten Aufnahmeparameterwertes in der wenigstens einen diagnostisch relevanten Schnittebene erfasst werden kann. Mit anderen Worten hängt der erste Aufnahmeparameterwert direkt von der Ausrichtung der wenigstens einen diagnostisch relevanten Schnittebene ab.

Insbesondere kann der erste Aufnahmeparameterwert derart von der Ausrichtung abgeleitet werden, dass basierend auf dem ersten Aufnahmeparameterwert die Gradientensteuereinheit die Gradientenspule derart ansteuern kann, dass in der diagnostisch relevanten Schnittebene das Magnetfeld beim Erfassen des zweidimensionalen Schnittbildes konstant ist. Insbesondere kann der erste Aufnahmeparameterwert derart abgeleitet werden, dass die Gradientensteuereinheit basierend auf dem ersten Aufnahmeparameterwert die wenigstens eine Gradientenspule derart ansteuert, dass ein Magnetfeldgradient senkrecht auf die diagnostisch relevante Schnittebene ausgerichtet ist.

Die Erfinder haben erkannt, dass basierend auf der Ausrichtung der diagnostisch relevanten Schnittebene der erste Aufnahmeparameterwert bestimmt werden kann, mit welchem das zweidimensionale Schnittbild der diagnostisch relevanten Schnittebene erfasst werden kann. Das Erfassen des zweidimensionalen Schnittbildes der diagnostisch relevanten Schnittebene, insbesondere das Ansteuern des MRT Systems zum Erfassen des zweidimensionalen Schnittbildes, kann somit über den Aufnahmeparameter direkt von der Ausrichtung abhängen.

Nach einem weiteren Aspekt der Erfindung umfasst das Verfahren außerdem einen Verfahrensschritt eines Erfassens des zweidimensionalen Schnittbildes mit dem Magnet-Resonanz-Tomographie System in Abhängigkeit des wenigstens einen ersten Aufnahmeparameterwertes und einen Verfahrensschritt eines Bereitstellens des zweidimensionalen Schnittbildes.

Bei dem Erfassen des zweidimensionalen Schnittbildes ist das Untersuchungsobjekt in dem Magnetfeld angeordnet. Das Magnetfeld setzt sich zusammen aus dem homogenen Grundmagnetfeld und dem oben beschriebenen Magnetfeldgradienten, der dem Grundmagnetfeld überlagert ist. Die Protonen-Spins des Untersuchungsobjektes sind entlang des Magnetfeldes ausgerichtet und präzedieren mit einer Larmor-Frequenz, die von einer Magnetfeldstärke am Ort des Spins abhängt. Die Spins können durch Einstrahlen eines Anregungspulses angeregt werden. Dabei werden die Spins angeregt, die mit der Frequenz des Anregungspulses präzedieren. Durch das Anregen präzedieren die angeregten Spins in Phase. Außerdem werden die Spins beim Anregen relativ zu dem Magnetfeld gekippt. Nach Ende des Anregungspulses relaxieren die Spins in dem Magnetfeld in Abhängigkeit eines sie umgebenden Materials. Das heißt, die Spins dephasieren und richten sich wieder entlang des Magnetfeldes aus. Dabei senden die Spins ein elektromagnetisches Signal aus, welches mit einer Antenne bzw. Spule ausgelesen bzw. erfasst werden kann. Insbesondere wird eine Geschwindigkeit der Relaxation erfasst. Die Geschwindigkeit hängt von dem Material ab.

Beim Erfassen des zweidimensionalen Schnittbildes der diagnostisch relevanten Ebene wird der Magnetfeldgradient mit dem ersten Aufnahmeparameterwert derart angepasst, dass die Spins in der diagnostisch relevanten Schnittebene (wenigstens näherungsweise) mit der Frequenz des Anregungspulses präzedieren. Außerdem wird beim Erfassen des zweidimensionalen Schnittbildes das elektromagnetische Signal nach dem Anregen der Spins in der diagnostisch relevanten Schnittebene beim Relaxieren ausgelesen bzw. erfasst. Außerdem wird beim Erfassen des zweidimensionalen Schnittbildes, das zweidimensionale Schnittbild aus dem erfassten Signal rekonstruiert.

In dem Verfahrensschritt des Bereitstellens des zweidimensionalen Schnittbildes kann das zweidimensionale Schnittbild mit einer Schnittstelle bereitgestellt werden.

Insbesondere kann das zweidimensionale Schnittbild beim Bereitstellen des zweidimensionalen Schnittbildes in ein PACS geladen werden bzw. dort gespeichert werden. Mit anderen Worten kann das zweidimensionale Schnittbild in einer Datenbank gespeichert werden.

Alternativ oder zusätzlich kann in dem Verfahrensschritt des Bereitstellens des zweidimensionalen Schnittbildes das zweidimensionale Schnittbild dem medizinischen Bedienpersonal angezeigt werden. Insbesondere kann das Schnittbild mittels einer Anzeigeeinheit beispielsweise einem Bildschirm oder einem Monitor angezeigt werden.

Die Erfinder haben erkannt, dass in Abhängigkeit der Ausrichtung der wenigstens einen diagnostisch relevanten Schnittebene das zweidimensionale Schnittbild erfasst und bereitgestellt werden kann. Insbesondere kann das zweidimensionale Schnittbild dem medizinischen Bedienpersonal bereitgestellt werden. Insbesondere kann das medizinische Bedienpersonal eine Diagnose oder eine Untersuchung basierend auf dem zweidimensionalen Schnittbild erstellen.

Nach einem weiteren Aspekt der Erfindung umfasst das Verfahren einen Verfahrensschritt eines Bestimmens einer Ausdehnung eines dreidimensionalen Volumenbildes senkrecht zu der diagnostisch relevanten Schnittebene. Dabei wird das dreidimensionale Volumenbild von der diagnostisch relevanten Schnittebene und der Ausdehnung aufgespannt. Das Verfahren umfasst außerdem einen Verfahrensschritt eines Bereitstellens wenigstens eines zweiten Aufnahmeparameterwertes zum Steuern eines Magnet-Resonanz-Tomographie Systems zum Erfassen des dreidimensionalen Volumenbildes. Dabei wird der wenigstens eine zweite Aufnahmeparameterwert von der Ausrichtung der wenigstens einen diagnostisch relevanten Schnittebene und der Ausdehnung abgeleitet. Das Verfahren umfasst außerdem einen Verfahrensschritt eines Erfassens des dreidimensionalen Volumenbildes mit dem Magnet-Resonanz-Tomographie System in Abhängigkeit des wenigstens einen zweiten Aufnahmeparameterwertes. Das Verfahren umfasst außerdem einen Verfahrensschritt eines Bereitstellens des dreidimensionalen Volumenbildes.

Das dreidimensionale Volumenbild ist eine dreidimensionale Darstellung bzw. Abbildung des wenigstens einen Teils des Herzens, die die diagnostisch relevante Schnittebene umfasst. Mit anderen Worten ist das zweidimensionale Schnittbild der diagnostisch relevanten Schnittebene von dem dreidimensionalen Volumenbild umfasst. Das dreidimensionale Volumenbild weist dabei eine Ausdehnung senkrecht zu der diagnostisch relevanten Schnittebene auf. Dabei kann die diagnostisch relevante Schnittebene an einer beliebigen Position innerhalb des dreidimensionalen Volumenbildes angeordnet sein. Das dreidimensionale Volumenbild wird dabei von der diagnostisch relevanten Schnittebene und der Ausdehnung aufgespannt. Mit anderen Worten wird der Bereich des Herzens, der in dem dreidimensionalen Volumenbild abgebildet ist, durch die diagnostisch relevante Schnittebene und die Ausdehnung senkrecht zu dieser Schnittebene beschrieben.

Das dreidimensionale Volumenbild kann beispielsweise einen Verlauf eines Zuflusses oder Abflusses zu dem Herzen abbilden. Das dreidimensionale Volumenbild kann insbesondere dazu ausgebildet sein einen Fluss in dem Herzen in dem Volumenbild zu analysieren.

In dem Verfahrensschritt des Bestimmens der Ausdehnung wird die Ausdehnung des dreidimensionalen Volumenbildes senkrecht zu der diagnostisch relevanten Schnittebene bestimmt. Die Ausdehnung kann beispielsweise 0,1cm oder 0,5cm oder 1cm oder 2cm umfassen. Die Ausdehnung wird insbesondere in Abhängigkeit einer anatomischen Gegebenheit des abgebildeten Herzens bestimmt. Die anatomische Gegebenheit kann insbesondere in Abhängigkeit des dreidimensionalen Bilddatensatzes bestimmt werden. Insbesondere kann die anatomische Gegebenheit durch eine oder mehrere wie oben beschrieben bestimmte Landmarken bestimmt werden bzw. vorgegeben sein bzw. abhängen. Beispielsweise kann das dreidimensionale Volumenbild derart ausgedehnt sein, dass es den gesamten Aortenbogen abbildet. Dann hängt die Ausdehnung von der Größe des Aortenbogens senkrecht zu der diagnostisch relevanten Schnittebene ab. In dem Bespiel verläuft die diagnostisch relevante Schnittebene durch den Aortenbogen. Die Abhängigkeit von anderen Anatomien kann analog zu dem Beispiel übertragen werden.

In dem Verfahrensschritt des Bereitstellens des wenigstens einen zweiten Aufnahmeparameterwertes wird der zweite Aufnahmeparameterwert analog wie oben für den ersten Aufnahmeparameterwert beschrieben bereitgestellt. Der zweite Aufnahmeparameterwert kann insbesondere analog zu dem ersten Aufnahmeparameterwert ausgebildet sein.

Zusätzlich umfasst der zweite Aufnahmeparameterwert eine Abfolge von Frequenzen des Anregungspulses. Die Abfolge der Frequenzen ist derart ausgebildet, dass die Spins in verschiedenen Schnittebenen parallel zu der diagnostisch relevanten Schnittebene innerhalb des von dem dreidimensionalen Volumenbildes aufgespannten Bereiches angeregt werden. Insbesondere ist eine der Frequenzen derart ausgebildet, dass die Spins in der diagnostisch relevante Schnittebene angeregt werden.

Alternativ umfasst das zweite Aufnahmeparameterwert eine Abfolge von Stärken des Magnetfeldgradienten. Die Abfolge der Stärken des Magnetfeldgradienten ist derart ausgebildet, dass bei konstanter Frequenz des Anregungspulses die Spins in verschiedene Schnittebenen innerhalb des dreidimensionalen Volumenbildes angeregt werden können.

In dem Verfahrensschritt des Erfassens des dreidimensionalen Volumenbildes wird das dreidimensionale Volumenbild in Abhängigkeit des zweiten Aufnahmeparameterwertes mit dem Magnet-Resonanz-Tomographie System erfasst. Dabei wird für jede Frequenz der Abfolge von Frequenzen oder für jede Stärke des Magnetfeldgradienten der Abfolge von Stärken des Magnetfeldgradienten ein Signal erfasst. Aus den Signalen kann das dreidimensionale Volumenbild wird aus der Magnet-Resonanz-Tomographie Bildgebung bekannt rekonstruiert werden.

In dem Verfahrensschritt des Bereitstellens des dreidimensionalen Volumenbildes wird das dreidimensionale Volumenbild wie oben für das zweidimensionale Schichtbild beschrieben bereitgestellt.

Die Erfinder haben erkannt, dass in manchen Fällen eine zweidimensionale Abbildung der diagnostisch relevanten Schnittebene für eine Diagnose oder Untersuchung nicht ausreichend ist. Die Erfinder haben erkannt, dass dann ausgehend von der diagnostisch relevanten Schnittebene eine Ausdehnung eines dreidimensionalen Volumenbildes bestimmt werden kann, welches für die Diagnose oder die Untersuchung geeignet ist.

Nach einem weiteren Aspekt der Erfindung basiert die trainierte Funktion auf einem neuronalen Faltungsnetzwerk und/oder einem U-Netzwerk.

Das neuronale Faltungsnetzwerk kann auch als "convolutional neural network" (Akronym: CNN) bezeichnet werden. Das U-Netzwerk kann auch als "U-net" bezeichnet werden.

Das Faltungsnetzwerk bzw. das U-Netzwerk umfassen einen Eingang und einen Ausgang. In den Eingang können Eingangsdaten eingegeben werden, auf die die trainierte Funktion bzw. das Faltungsnetzwerk bzw. das U-Netzwerk angewendet werden können. Die Eingangsdaten können dabei insbesondere den dreidimensionalen Bilddatensatz umfassen.

Beim Anwenden der trainierten Funktion auf die Eingangsdaten werden Ausgangsdaten erzeugt. Die Ausgangsdaten können insbesondere die Position der wenigstens einen Landmarke, insbesondere die Wahrscheinlichkeitsverteilung, umfassen.

Das Faltungsnetzwerk bzw. das U-Netzwerk umfassen eine Mehrzahl von Schichten (engl.: Layer). Die Schichten sind insbesondere in einer Reihenfolge angeordnet. Die Eingangsdaten durchlaufen das Faltungsnetzwerk bzw. das U-Netzwerk in der Reihenfolge. Jede der Schichten umfasst eine Mehrzahl von Neuronen bzw. Knoten. Die Neuronen der Schichten können untereinander verknüpft sein. Mit anderen Worten kann ein Neuron mit einem oder mehr Neuronen der vorhergehenden Schicht verknüpft sein. Insbesondere können die vorhergehenden Neuronen einen Wert des verknüpften Neurons in der folgenden Schicht beeinflussen. Insbesondere kann eine Verknüpfung gewichtet sein. Das Gewicht der Verknüpfung bestimmt den Einfluss, den das vorhergehende Neuron auf das mit der Verknüpfung verknüpfte Neuron in der folgenden Schicht hat. Die Gewichte können beim Trainieren des Netzwerkes bzw. beim Trainieren der trainierten Funktion angepasst bzw. gelernt bzw. trainiert werden.

Das Faltungsnetzwerk bzw. das U-Netzwerk umfassen wenigstens einen Eingangsschicht und eine Ausgangsschicht.

Die Anzahl der von der Eingangsschicht umfassten Neuronen kann insbesondere der Anzahl der Voxel in der Voxelmatrix des dreidimensionalen Bilddatensatzes entsprechen.

In Ausführungen der Erfindung kann die Ausgangsschicht wenigstens ein Neuron umfassen. Alternativ kann die Anzahl der Neuronen in der Ausgangsschicht der Anzahl von Voxeln in der Voxelmatrix des dreidimensionalen Bilddatensatzes entsprechen, wenn durch Anwenden der trainierten Funktion die Wahrscheinlichkeitsverteilung bestimmt wird. In Ausführungen der Erfindung kann für jede Landmarke eine Wahrscheinlichkeitsverteilung durch Anwenden der trainierten Funktion bestimmt werden. Dann kann die Anzahl der Neuronen der Ausgangsschicht dem Produkt der Anzahl von Voxeln in der Voxelmatrix des dreidimensionalen Bilddatensatzes und der Anzahl von Landmarken entsprechen.

Das Faltungsnetzwerk kann eine oder mehr Faltungsschichten (engl.: convolutional layer) umfassen. Das Faltungsnetzwerk kann außerdem eine oder mehr Entfaltungsschichten (engl.: deconvolutional layer) umfassen. Insbesondere kann die eine oder mehrere Faltungsschichten in der Reihenfolge vor der einen oder mehreren Entfaltungsschichten angeordnet sein. Das Faltungsnetzwerk kann außerdem eine oder mehrere Pooling-Schichten umfassen.

Das U-Netzwerk kann insbesondere ein wie oben beschrieben ausgebildetes Faltungsnetzwerk umfassen. Insbesondere können die Schichten des Faltungsnetzwerkes in dem U-Netzwerk u-förmig miteinander verknüpft sein. Mit anderen Worten ist in dem U-Netzwerk die Eingangsschicht mit der Ausgangsschicht verknüpft. Die Schicht, die auf die Eingangsschicht folgt ist mir der vorletzten Schicht, also mit der Schicht vor der Ausgangsschicht, verknüpft usw. Über die Verknüpfung können Information von einer Schicht in der mit dieser Schicht verknüpften Schicht berücksichtigt werden.

Die Erfinder haben erkannt, dass ein Faltungsnetzwerk oder ein U-Netzwerk besonders geeignet sind, um die Position der wenigstens einen Landmarke in den dreidimensionalen Bilddaten zu bestimmen. Insbesondere ist ein Faltungsnetzwerk oder ein U-Netzwerk besonders geeignet die Wahrscheinlichkeitsverteilung der wenigstens einen Landmarke zu bestimmen.

Die Erfindung betrifft außerdem ein computerimplementiertes Verfahren zum Bereitstellen einer trainierten Funktion zum Bestimmen einer Position wenigstens einer Landmarke in einem dreidimensionalen Magnet-Resonanz-Tomographie Bilddatensatz. Das Verfahren umfasst einen Verfahrensschritt eines Empfangens von wenigstens einem dreidimensionalen Trainings-Bilddatensatz. Das Verfahren umfasst außerdem einen Verfahrensschritt eines Empfangens von wenigstens einem annotierten dreidimensionalen Trainings-Bilddatensatz. Dabei basiert der annotierte dreidimensionale Trainings-Bilddatensatz auf dem dreidimensionalen Trainings-Bilddatensatz. Dabei ist in dem annotierten dreidimensionalen Trainings-Bilddatensatz die Position der wenigstens einen Landmarke annotiert. Das Verfahren umfasst außerdem einen Verfahrensschritt eines Trainierens einer Funktion in Abhängigkeit des dreidimensionalen Trainings-Bilddatensatzes und des annotierten dreidimensionalen Trainings-Bilddatensatzes. Das Verfahren umfasst außerdem einen Verfahrensschritt eines Bereitstellens der trainierten Funktion.

Der dreidimensionale (MRT) Trainings-Bilddatensatz ist insbesondere wie der oben beschriebene dreidimensionale (MRT) Bilddatensatz ausgebildet.

In den Verfahrensschritten des Empfangens des dreidimensionalen Trainings-Bilddatensatzes und des Empfangens des annotierten dreidimensionalen Trainings-Bilddatensatzes können die Bilddatensätze von einer Datenbank mit einer Trainings-Schnittstelle empfangen werden. Die Datenbank kann insbesondere auf einem Server gespeichert sein. In Ausführungen der Erfindung kann die Datenbank ein PACS sein.

In dem annotierten dreidimensionalen Trainings-Bilddatensatz ist insbesondere die Position der wenigstens einen Landmarke annotiert bzw. eingezeichnet bzw. markiert. Insbesondere kann der annotierte dreidimensionale Trainings-Bilddatensatz dem dreidimensionalen Trainings-Bilddatensatz entsprechen, wobei in dem annotierten dreidimensionalen Trainings-Bilddatensatz die Position der wenigstens einen Landmarke eingezeichnet bzw. markiert bzw. annotiert ist. Insbesondere können die Positionen von mehr als einer Landmarke in dem annotierten dreidimensionalen Trainings-Bilddatensatz annotiert sein. Alternativ können die Positionen der verschiedenen Landmarken in Kopien des dreidimensionalen Trainings-Bilddatensatzes annotiert sein. Mit anderen Worten kann in jeder Kopie die Position einer anderen Landmarke annotiert sein.

In Ausführungen der Erfindung kann in dem annotierten dreidimensionalen Trainings-Bilddatensatz die Position der wenigstens einen Landmarke in Form eines oder mehrerer Bereiche eingezeichnet bzw. annotiert sein. Die verschiedenen Bereiche können angeben, wo die wenigstens eine Landmarke in dem annotierten dreidimensionalen Trainings-Bilddatensatz angeordnet sein kann. Die verschiedenen Bereiche betreffen dabei verschiedene Wahrscheinlichkeiten, mit denen die wenigstens eine Landmarke innerhalb des entsprechenden Bereiches angeordnet ist.

In alternativen Ausführungen der Erfindung kann der annotierte dreidimensionale Trainings-Bilddatensatz wenigstens eine dreidimensionale Koordinate umfassen. Die dreidimensionale Koordinate gibt dabei die Position der wenigstens einen Landmarke in der Voxelmatrix des dreidimensionalen Trainings-Bilddatensatzes an.

In dem Verfahrensschritt des Trainierens wird die Funktion auf den dreidimensionalen Trainings-Bilddatensatz angewendet. Dabei wird die Position der wenigstens einen Landmarke bestimmt. Diese derart bestimmte Position wird mit der Position der Landmarke gemäß dem annotierten dreidimensionalen Trainings-Bilddatensatz verglichen. Bei einer Abweichung wird die Funktion derart angepasst, dass die bestimmte Position näher an der Position gemäß dem annotierten dreidimensionalen Trainings-Bilddatensatz liegt. Dies kann iterativ wiederholt werden, bis die beiden Positionen einander entsprechen oder die Abweichung zwischen den Positionen einen Grenzwert unterschreitet. Mit anderen Worten kann die Abweichung iterativ durch Anpassen bzw. Trainieren der Funktion minimiert werden.

Insbesondere kann der Verfahrensschritt des Trainierens für eine Mehrzahl von dreidimensionalen Trainings-Bilddatensätzen und entsprechenden annotierten dreidimensionalen Trainings-Bilddatensätzen wiederholt werden.

Insbesondere kann die Funktion so lange trainiert werden, bis ein Abbruchkriterium erreicht ist. Das Abbruchkriterium kann beispielsweise eine maximale Anzahl von Trainingsdurchläufen bzw. Iterationen sein. Alternativ kann das Abbruchkriterium eine maximale Anzahl von dreidimensionalen Trainings-Bilddatensätzen sein, auf welchen die Funktion trainiert wird. Alternativ oder zusätzlich kann das Abbruchkriterium ein Unterschreiten einer maximalen Abweichung der mit der Funktion bestimmten Position und der in dem annotierten dreidimensionalen Trainings-Bilddatensatz vorgegebenen Position der wenigstens einen Landmarke sein.

In dem Verfahrensschritt des Bereitstellens der trainierten Funktion wird die trainierte Funktion derart bereitgestellt, dass sie wie oben beschrieben auf einen dreidimensionalen Bilddatensatz angewendet werden kann.

Die Erfinder haben erkannt, dass die Funktion mittels überwachten Lernens trainiert werden kann. Die Erfinder haben erkannt, dass ausreichend Trainingsdaten zu Verfügung stehen. Insbesondere kann bei den annotierten dreidimensionalen Trainings-Bilddatensätzen auf eine Erfahrung zurückgegriffen werden. Mit anderen Worten ist bereits eine große Menge an (annotierten) dreidimensionalen Bilddatensätzen vorhanden, die zum Trainieren der Funktion genutzt werden können.

Nach einem Aspekt der Erfindung wird der annotierte dreidimensionale Trainings-Bilddatensatz durch manuelles Annotieren des dreidimensionalen Trainings-Bilddatensatz erstellt.

Insbesondere kann der dreidimensionale Trainings-Bilddatensatz von einem erfahrenen medizinischen Bedienpersonal annotiert werden. Dabei kann das medizinische Bedienpersonal insbesondere eine medizinisch-technische Radiologieassistenz (Akronym: MTRA) und/oder ein Radiologe bzw. eine Radiologin sein.

Das medizinische Bedienpersonal kann in dem dreidimensionalen Trainings-Bilddatensatz eine Position der wenigstens einen Landmarke manuell einzeichnen bzw. annotieren bzw. markieren.

Dabei kann das medizinische Bedienpersonal einen Voxel in dem dreidimensionalen Trainings-Bilddatensatz markieren, in dem die wenigstens eine Landmarke abgebildet ist. Insbesondere kann die Position der wenigstens einen Landmarke bzw. der annotierte dreidimensionale Trainings-Bilddatensatz die Koordinate des markierten Voxels umfassen.

In alternativen Ausführungen der Erfindung kann das medizinische Bedienpersonal einen oder mehrere Bereiche als mögliche Position der wenigstens einen Landmarke in dem dreidimensionalen Trainings-Bilddatensatz einzeichnen. Die Bereiche können dabei eine Unsicherheit der Position der wenigstens einen Landmarke angeben. Mit anderen Worten kann mit einem Bereich der Bereich in dem dreidimensionalen Trainings-Bilddatensatz angegeben werden, in dem die Landmarke mit einer bestimmten Wahrscheinlichkeit positioniert ist. Verschiedene Bereiche können sich dabei auf verschiedenen Wahrscheinlichkeiten beziehen. Dabei kann mit abnehmender Wahrscheinlichkeit der entsprechende Bereich immer größer werden.

In Ausführungen der Erfindung kann der dreidimensionale Trainings-Bilddatensatz mehrfach von verschiedenem medizinischen Bedienpersonal annotiert werden. Insbesondere kann dann der annotierte dreidimensionale Trainings-Bilddatensatz ein Mittel der verschiedenen Annotierungen sein. Alternativ kann jeder der annotierten dreidimensionalen Trainings-Bilddatensätze einzeln zum Trainieren der Funktion genutzt werden.

Die Erfinder haben erkannt, dass das medizinische Bedienpersonal den dreidimensionalen Trainings-Bilddatensatz manuell annotieren kann. Auf diese Weise kann eine Erfahrung des medizinischen Bedienpersonals beim Trainieren der Funktion berücksichtigt bzw. genutzt werden. Insbesondere kann auf diese Weise eine Vielzahl von annotierten dreidimensionalen Trainings-Bilddatensätzen erzeugt bzw. generiert werden.

Die Erfindung betrifft außerdem ein Bestimmungssystem zum Bestimmen einer Ausrichtung wenigstens einer diagnostisch relevanten Schnittebene für eine Herzbildgebung in einem dreidimensionalen Magnet-Resonanz-Tomographie Bilddatensatz. Das Bestimmungssystem umfasst eine Schnittstelle und eine Recheneinheit. Dabei ist die Schnittstelle zum Bereitstellen des dreidimensionalen Bilddatensatzes ausgebildet. Dabei ist die Recheneinheit zum Anwenden einer trainierten Funktion auf den dreidimensionalen Bilddatensatz ausgebildet. Dabei wird eine Position wenigstens einer Landmarke bestimmt. Dabei ist die Recheneinheit außerdem zum Bestimmen der Ausrichtung der wenigstens einen diagnostisch relevanten Schnittebene in Abhängigkeit der wenigstens einen Landmarke ausgebildet. Dabei ist die Schnittstelle außerdem zum Bereitstellen der Ausrichtung der wenigstens einen diagnostisch relevanten Schnittebene ausgebildet.

Eine solches Bestimmungssystem kann insbesondere dazu ausgebildet sein das zuvor beschriebene Verfahren zum Bestimmen einer Ausrichtung wenigstens einer diagnostisch relevanten Schnittebene für eine Herzbildgebung in einem dreidimensionalen Magnet-Resonanz-Tomographie Bilddatensatz und seine Aspekte auszuführen. Das Bestimmungssystem ist dazu ausgebildet dieses Verfahren und seine Aspekte auszuführen, indem die Schnittstelle und die Recheneinheit ausgebildet sind, die entsprechenden Verfahrensschritte auszuführen.

Die Erfindung betrifft auch ein Magnet-Resonanz-Tomographie System. Das Magnet-Resonanz-Tomographie System umfasst ein oben beschriebenes Bestimmungssystem. Dabei ist das Magnet-Resonanz-Tomographie System zum Erfassen des dreidimensionalen Bilddatensatzes und/oder eines zweidimensionalen Schnittbildes ausgebildet.

Das MRT System umfasst bevorzugt ein medizinisches und/oder diagnostisches MRT System, das zu einem Erfassen von medizinischen und/oder diagnostischen MRT Aufnahmen bzw. Bilddaten bzw. dreidimensionalen Bilddatensätzen und/oder zweidimensionalen Schnittbildern, eines oben beschriebenen Untersuchungsobjektes ausgelegt und/oder ausgebildet ist. Das MRT System umfasst hierzu eine Scannereinheit. Die Scannereinheit des MRT Systems umfasst bevorzugt eine Detektoreinheit, insbesondere eine Magneteinheit, zum Erfassen des dreidimensionalen Bilddatensatzes und/oder des zweidimensionalen Schnittbildes. Vorteilhafterweise umfasst hierbei die Scannereinheit, insbesondere die Magneteinheit, einen Grundmagneten, eine Gradientenspuleneinheit und eine System-Spule bzw. Hochfrequenz-Antennen-Einheit. Die System-Spule ist fest innerhalb der Scannereinheit angeordnet und zur Aussendung eines Anregungspulses ausgelegt bzw. ausgebildet. Zum Erfassen des Magnetresonanzsignals bzw. Radiosignals bzw. Signals weist das MRT System wenigstens eine Lokalspule auf, die um einen zu untersuchenden Bereich des Untersuchungsobjektes angeordnet werden kann.

Der Grundmagnet der Scannereinheit ist zur Erzeugung eines homogenen Grundmagnetfelds mit einer definierten und/oder bestimmten Magnetfeldstärke, wie beispielsweise mit einer definierten und/oder bestimmten Magnetfeldstärke von 3 T oder 1,5 T usw., ausgebildet. Insbesondere ist der Grundmagnet zur Erzeugung eines starken, konstanten und homogenen Grundmagnetfelds ausgebildet. Das homogene Grundmagnetfeld ist bevorzugt innerhalb eines Aufnahmebereiches für das Untersuchungsobjekt des MRT Systems angeordnet bzw. vorzufinden. Die Gradientenspuleneinheit ist zu einer Erzeugung von Magnetfeldgradienten, die für eine Ortskodierung während einer Bildgebung verwendet werden, ausgebildet.

Der Aufnahmebereich ist zu einer Aufnahme des Untersuchungsobjektes, insbesondere des zu untersuchenden Bereichs des Untersuchungsobjektes, für eine medizinische MRT Untersuchung ausgelegt und/oder ausgebildet. Beispielsweise ist hierzu der Aufnahmebereich zylinderförmig ausgebildet und/oder zylinderförmig von der Scannereinheit umgeben. Hierzu weist die Scannereinheit eine den Aufnahmebereich zumindest teilweise umgebende Umhausung der Gehäuseeinheit auf. Die den Aufnahmebereich umgebende Umhausung kann hierbei auch einteilig und/ oder einstückig mit der dem Aufnahmebereich zugewandten Seite der System-Spule der Scannereinheit ausgebildet sein oder auch separat zur System-Spule der Scannereinheit ausgebildet sein.

Innerhalb des Aufnahmebereichs ist bevorzugt ein Field of View (FOV) und/oder ein Isozentrum des MRT Systems angeordnet. Das FOV umfasst bevorzugt einen Erfassungsbereich des MRT Systems, innerhalb dessen die Bedingungen für eine Erfassung einer MRT Aufnahme, insbesondere MRT Bilddaten, vorliegen, wie beispielsweise ein homogenes Grundmagnetfeld. Das Isozentrum des MRT Systems umfasst bevorzugt den Bereich und/oder Punkt innerhalb des MRT Systems, der die optimalen und/oder idealen Bedingungen für die Erfassung einer MRT Aufnahme, insbesondere MRT Bilddaten, aufweist. Insbesondere umfasst das Isozentrum den homogensten Grundmagnetfeldbereich innerhalb des MRT Systems.

Während einer MRT Untersuchung befindet sich das Untersuchungsobjekt auf einer Liege liegend innerhalb des Aufnahmebereichs des MRT Systems. Dagegen befindest sich ein medizinisches Bedienpersonal bzw. der Nutzer in einem Kontrollraum, der getrennt von einem Untersuchungsraum, in dem das MRT System angeordnet ist, ist.

Die Erfindung betrifft auch ein Computerprogrammprodukt mit einem Computerprogramm sowie ein computerlesbares Medium. Eine weitgehend softwaremäßige Realisierung hat den Vorteil, dass auch schon bisher verwendete Bestimmungssysteme auf einfache Weise durch ein Software-Update nachgerüstet werden können, um auf die beschriebene Weise zu arbeiten. Ein solches Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Insbesondere betrifft die Erfindung auch ein Computerprogrammprodukt mit einem Computerprogramm, welches direkt in einen Speicher eines Bestimmungssystems ladbar ist, mit Programmabschnitten, um alle Schritte des oben beschriebenen Verfahrens zum Bestimmen einer Ausrichtung wenigstens einer diagnostisch relevanten Schnittebene für eine Herzbildgebung in einem dreidimensionalen Magnet-Resonanz-Tomographie Bilddatensatz und seine Aspekte auszuführen, wenn die Programmabschnitte von dem Bestimmungssystem ausgeführt werden.

Insbesondere betrifft die Erfindung ein computerlesbares Speichermedium, auf welchem von einem Bestimmungssystem lesbare und ausführbare Programmabschnitte gespeichert sind, um alle Schritte des oben beschriebenen Verfahrens zum Bestimmen einer Ausrichtung wenigstens einer diagnostisch relevanten Schnittebene für eine Herzbildgebung in einem dreidimensionalen Magnet-Resonanz-Tomographie Bilddatensatz und seine Aspekte auszuführen, wenn die Programmabschnitte von dem Bestimmungssystem ausgeführt werden.

Die Erfindung betrifft außerdem ein Trainingssystem zum Bereitstellen einer trainierten Funktion zum Bestimmen einer Position wenigstens einer Landmarke in einem dreidimensionalen Magnet-Resonanz-Tomographie Bilddatensatz. Das Trainingssystem umfasst eine Trainings-Schnittstelle und eine Trainings-Recheneinheit. Die Trainings-Schnittstelle ist zum Empfangen wenigstens eines dreidimensionalen Trainings-Bilddatensatzes ausgebildet. Die Trainings-Schnittstelle ist außerdem zum Empfangen wenigstens eines annotierten dreidimensionalen Trainings-Bilddatensatzes ausgebildet. Dabei basiert der annotierte dreidimensionale Trainings-Bilddatensatz auf dem dreidimensionalen Trainings-Bilddatensatz. Dabei ist in dem annotierten dreidimensionalen Trainings-Bilddatensatz die Position der wenigstens einen Landmarke annotiert. Die Trainings-Recheneinheit ist zum Trainieren einer Funktion in Abhängigkeit des dreidimensionalen Trainings-Bilddatensatzes und des annotierten dreidimensionalen Trainings-Bilddatensatzes ausgebildet. Die Trainings-Schnittstelle ist außerdem zum Bereitstellen der trainierten Funktion ausgebildet.

Eine solches Trainingssystem kann insbesondere dazu ausgebildet sein das zuvor beschriebene Verfahren zum Bestimmen einer Position wenigstens einer Landmarke in einem dreidimensionalen Magnet-Resonanz-Tomographie Bilddatensatz und seine Aspekte auszuführen. Das Trainingssystem ist dazu ausgebildet dieses Verfahren und seine Aspekte auszuführen, indem die Trainings-Schnittstelle und die Trainings-Recheneinheit ausgebildet sind, die entsprechenden Verfahrensschritte auszuführen.

Die Erfindung betrifft auch ein Trainings-Computerprogrammprodukt mit einem Trainings-Computerprogramm sowie ein computerlesbares Trainings-Medium. Eine weitgehend softwaremäßige Realisierung hat den Vorteil, dass auch schon bisher verwendete Trainingssysteme auf einfache Weise durch ein Software-Update nachgerüstet werden können, um auf die beschriebene Weise zu arbeiten. Ein solches Trainings-Computerprogrammprodukt kann neben dem Trainings-Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Insbesondere betrifft die Erfindung auch ein Trainings-Computerprogrammprodukt mit einem Trainings-Computerprogramm, welches direkt in einen Trainings-Speicher eines Trainingssystems ladbar ist, mit Trainings-Programmabschnitten, um alle Schritte des oben beschriebenen Verfahrens zum Bestimmen einer Position wenigstens einer Landmarke in einem dreidimensionalen Magnet-Resonanz-Tomographie Bilddatensatz und seine Aspekte auszuführen, wenn die Trainings-Programmabschnitte von dem Trainingssystem ausgeführt werden.

Insbesondere betrifft die Erfindung ein computerlesbares Trainings-Speichermedium, auf welchem von einem Trainingssystem lesbare und ausführbare Trainings-Programmabschnitte gespeichert sind, um alle Schritte des oben beschriebenen Verfahrens zum Bestimmen einer Position wenigstens einer Landmarke in einem dreidimensionalen Magnet-Resonanz-Tomographie Bilddatensatz und seine Aspekte auszuführen, wenn die Programmabschnitte von dem Trainingssystem ausgeführt werden.

Die oben beschriebenen Eigenschaften, Merkmale und Vorteile dieser Erfindung werden klarer und verständlicher im Zusammenhang mit folgenden Figuren und ihren Beschreibungen. Dabei sollen die Figuren und Beschreibungen die Erfindung und ihre Ausführungsformen in keiner Weise einschränken.

In verschiedenen Figuren sind gleiche Komponenten mit korrespondierenden Bezugszeichen versehen. Die Figuren sind in der Regel nicht maßstabsgetreu.

Es zeigen
- Fig. 1: ein erstes Ausführungsbeispiel eines computerimplementierten Verfahrens zum Bestimmen einer Ausrichtung wenigstens einer diagnostisch relevanten Schnittebene für eine Herzbildgebung in einem dreidimensionalen Magnet-Resonanz-Tomographie Bilddatensatz,
- Fig. 2: ein zweites Ausführungsbeispiel eines computerimplementierten Verfahrens zum Bestimmen einer Ausrichtung wenigstens einer diagnostisch relevanten Schnittebene für eine Herzbildgebung in einem dreidimensionalen Magnet-Resonanz-Tomographie Bilddatensatz,
- Fig. 3: ein drittes Ausführungsbeispiel eines computerimplementierten Verfahrens zum Bestimmen einer Ausrichtung wenigstens einer diagnostisch relevanten Schnittebene für eine Herzbildgebung in einem dreidimensionalen Magnet-Resonanz-Tomographie Bilddatensatz,
- Fig. 4: ein viertes Ausführungsbeispiel eines computerimplementierten Verfahrens zum Bestimmen einer Ausrichtung wenigstens einer diagnostisch relevanten Schnittebene für eine Herzbildgebung in einem dreidimensionalen Magnet-Resonanz-Tomographie Bilddatensatz,
- Fig. 5: ein Ausführungsbeispiel eines computerimplementierten Verfahrens zum Bereitstellen einer trainierten Funktion zum Bestimmen einer Position wenigstens einer Landmarke in einem dreidimensionalen Magnet-Resonanz-Tomographie Bilddatensatz,
- Fig. 6: ein Bestimmungssystem zum Bestimmen einer Ausrichtung wenigstens einer diagnostisch relevanten Schnittebene für eine Herzbildgebung in einem dreidimensionalen Magnet-Resonanz-Tomographie Bilddatensatz,
- Fig. 7: ein Trainingssystem zum Bereitstellen einer trainierten Funktion zum Bestimmen einer Position wenigstens einer Landmarke in einem dreidimensionalen Magnet-Resonanz-Tomographie Bilddatensatz,
- Fig. 8: ein Magnet-Resonanz-Tomographie System.

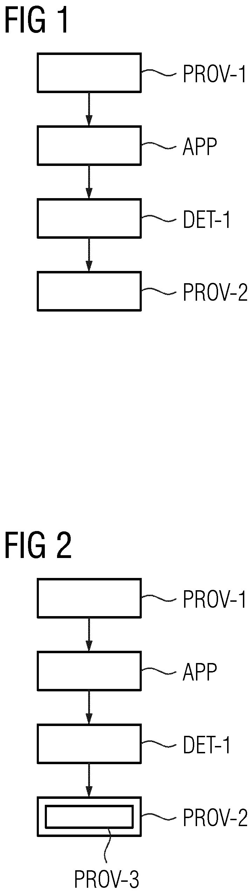

**Figur 1** zeigt ein erstes Ausführungsbeispiel eines computerimplementierten Verfahrens zum Bestimmen einer Ausrichtung wenigstens einer diagnostisch relevanten Schnittebene für eine Herzbildgebung in einem dreidimensionalen Magnet-Resonanz-Tomographie Bilddatensatz.

Der dreidimensionale Magnet-Resonanz-Tomographie (Akronym: MRT) Bilddatensatz bzw. dreidimensionale Bilddatensatz umfasst eine dreidimensionale Darstellung wenigstens eines Teils eines Untersuchungsobjektes. Das Untersuchungsobjekt ist dabei ein Mensch, insbesondere ein Patient, oder ein Tier. Insbesondere umfasst der dargestellte Teil des Untersuchungsobjektes das Herz bzw. wenigstens einen Teil des Herzens des Untersuchungsobjektes. Mit anderen Worten umfasst der dreidimensionale Bilddatensatz eine dreidimensionale Darstellung wenigstens eines Teils des Herzens des Untersuchungsobjektes.

Der dreidimensionale Bilddatensatz umfasst eine Mehrzahl von Voxeln, die in einer dreidimensionalen Voxelmatrix angeordnet sind. Jeder Voxel umfasst dabei einen Voxelwert.

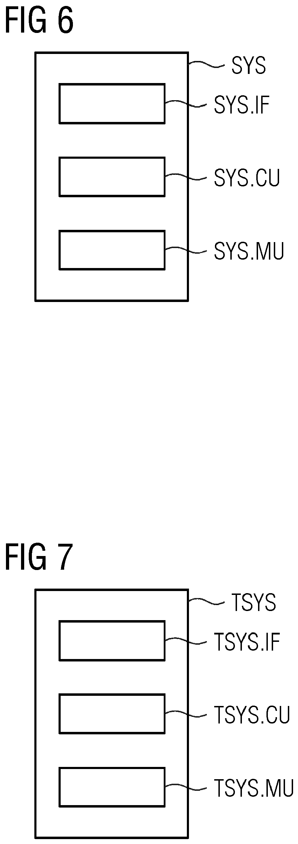

In Ausführungen der Erfindung kann der dreidimensionale Bilddatensatz einen Übersichtsscan wenigstens des Teils des Herzens umfassen. Der Übersichtsscan kann dabei mit einem in Figur 7 beschriebenen MRT System erfasst worden sein. Bei einem Übersichtsscan wird eine Bildqualität, insbesondere eine räumliche Auflösung und/oder ein Signal-zu-Rausch-Verhältnis, zu Gunsten einer verkürzten Aufnahmezeit reduziert.

In Ausführungen der Erfindung kann der dreidimensionale Bilddatensatz eine zweidimensionale Multischicht Aufnahme umfassen. Die zweidimensionale Multischicht Aufnahme umfasst eine Mehrzahl von zweidimensionalen Schnittbildern bzw. Schichtbildern des Herzens bzw. des Teils des Herzens. Die Mehrzahl der Schichtbilder ist insbesondere parallel untereinander bzw. zueinander ausrichtet. Diese Schichtbilder können räumlich hintereinander angeordnet werden bzw. "gestapelt" werden. Auf diese Weise wird eine "pseudo" dreidimensionale Darstellung des Herzens bzw. des Teils des Herzens erzeugt. Die einzelnen Schichtbilder tasten dabei das Herz bzw. den Teil des Herzens senkrecht zu der Bildebene der Schichtbilder ab.

Eine Schnittebene ist ein zweidimensionaler Schnitt durch den dreidimensionalen Bilddatensatz. In einem Schnittbild der Schnittebene kann der zweidimensionale Schnitt abgebildet bzw. dargestellt werden. Das Schnittbild kann mit einem in Figur 7 beschriebenen MRT System erfasst werden. Die diagnostisch relevante Schnittebene beschreibt insbesondere eine vordefinierte bzw. standardisierte Schnittebene durch das Herz. Mit anderen Worten ist in dem Schnittbild der diagnostisch relevanten Schnittebene eine vordefinierte bzw. standardisierte Darstellung eines zweidimensionalen Schnittes durch das Herz abgebildet bzw. dargestellt. Die vordefinierte bzw. standardisierte Darstellung gibt an, welche anatomischen Strukturen des Herzens wie und in welchem Zusammenhang dargestellt werden sollen. Anhand der Darstellung der diagnostisch relevanten Schnittebene in einem Schnittbild, kann eine Diagnose und/oder eine Untersuchung des Untersuchungsobjektes erstellt bzw. durchgeführt werden.

Die Ausrichtung der diagnostisch relevanten Schnittebene beschreibt die Ausrichtung relativ zu dem in einem Aufnahmebereich des MRT Systems positionierten Untersuchungsobjekt bzw. relativ zu dem MRT System.

Insbesondere beschreibt die Ausrichtung eine räumliche Lage bzw. räumliche Ausrichtung bzw. Angulation der diagnostisch relevanten Schnittebene im Raum. Die räumliche Lage kann beispielsweise durch eine Richtung eines Normalenvektors auf die diagnostisch relevante Schnittebene beschrieben sein. In Ausführungen kann die Ausrichtung zusätzlich eine Position der diagnostisch relevanten Schnittebene relativ zu dem Untersuchungsobjekt oder dem MRT System beschreiben. Die Position kann dabei beispielsweise durch Punkte, die innerhalb der diagnostisch relevanten Schnittebene liegen sollen vorgegeben sein. Mit anderen Worten kann die Ausrichtung eine Information über die räumliche Lage und in Ausführungen der Erfindung über die Position der diagnostisch relevanten Schnittebene umfassen.

In einem Verfahrensschritt eines Bereitstellens PROV-1 wird der dreidimensionale Bilddatensatz insbesondere mit einer Schnittstelle SYS.IF bereitgestellt. Dabei kann das Bereitstellen PROV-1 ein Abrufen oder ein Empfangen oder ein Erfassen des dreidimensionalen Bilddatensatzes umfassen.

Insbesondere kann der dreidimensionale Bilddatensatz mit dem MRT System erfasst werden. In Ausführungen kann der dreidimensionale Bilddatensatz von dem MRT System bereitgestellt werden.

Alternativ kann der dreidimensionale Bilddatensatz von einem Server, insbesondere von einer Datenbank auf dem Server, abgerufen bzw. empfangen werden. Der Server kann insbesondere ein lokaler Server oder ein Cloud-Server sein. Die Datenbank kann insbesondere ein Bildarchivierungs- und Kommunikationssystem (engl.: picture archiving and communication system, Akronym: PACS) sein.

In einem Verfahrensschritt eines Anwendens APP einer trainierten Funktion wird die trainierte Funktion auf den dreidimensionalen Bilddatensatz angewendet. Mit anderen Worten umfassen die Eingangsdaten der trainierten Funktion den dreidimensionalen Bilddatensatz. Dabei wird eine Position wenigstens einer Landmarke bestimmt.

Die trainierte Funktion kann dabei auf einem neuronalen Faltungsnetzwerk (engl.: convolutional neural network, Akronym: CNN) und/oder einem U-Netzwerk (engl.: U-net) basieren.

In Ausführungen der Erfindung kann jeweils die Position von mehr als einer Landmarke in dem dreidimensionalen Bilddatensatz bestimmt werden.

Die wenigstens eine Landmarke betrifft dabei eine Anatomie des Herzens. Die wenigstens eine Landmarke kann beispielsweise eine der folgenden Landmarken sein: Apex, Mitralklappe, Aortenklappe, Pulmonalklappe, Trikuspidalklappe.

In Ausführungen der Erfindung kann die wenigstens eine Landmarke eine der folgenden Landmarken sein:
- Links Ventrikulärer Apex (engl.: left ventricular apex, Akronym: LV apex)
- Rechts Ventrikulärer Apex (engl.: right ventricular apex, Akronym: RV apex)
- Rechtes Ventrikel Ausdehnung (engl.: right ventricle extent, Akronym: RV extent)
- Mitralklappe, Zentrum (engl.: mitral valve, midpoint, Akronym: MV midpoint)
- Aortenklappe, Zentrum (engl.: aortic valve, midpoint, Akronym: AV midpoint)
- Trikuspidalklappe, Zentrum (engl.: tricuspid valve, midpoint, Akronym: TV midpoint)
- Pulmonalklappe, Zentrum (engl.: pulmonary valve, midpoint, Akronym: PA center point)
- Aortenbogen (engl.: aortic arch)
- Absteigende Aorta (engl.: descending aorta)
- Vier Punkte auf der Gefäßwand der Aortenwurzel (anterior, posterior, links, rechts) (engl.: aortic bulb)
- Vier Punkte auf der Gefäßwand der Pulmonalarterie (links, rechts, superior, inferior) an einer geeigneten Stelle für eine Flussmessung der Pulmonalarterie (engl.: pulmonary artery flow plane)
- Vier Punkte auf dem Annulus der Pulmonalklappe (links, rechts, superior, inferior) (engl.: pulmonary valve insertion)
- Vier Punkte auf dem Annulus der Mitralklappe (septal, lateral, superior, inferior) (engl.: mitral valve insertion)
- Vier Punkte auf dem Annulus der Trikuspidalklappe (septal, lateral, superior, inferior) (engl.: tricuspid valve insertion)
- Insertionspunkte des rechten Ventrikels (anterior, posterior) am linken Ventrikel (engl.: right ventricle insertion points)
- Mittellinie der absteigenden Aorta (engl.: descending aorta centerline)
- Mittellinie der aufsteigenden Aorta (engl.: ascending aorta centerline).

Die Position der wenigstens einen Landmarke kann durch ihre Position in der Voxelmatrix des dreidimensionalen Bilddatensatzes beschrieben werden. Mit anderen Worten kann die Position angeben, wo in der Voxelmatrix die wenigstens eine Landmarke abgebildet ist.

In Ausführungen der Erfindung kann die Position der wenigstens eine Landmarke in Form einer Wahrscheinlichkeitsverteilung (engl.: heatmap) angegeben werden. Dabei kann jedem Voxel der Voxelmatrix des dreidimensionalen Bilddatensatzes eine Wahrscheinlichkeit zugeordnet werden, die angibt, wie wahrscheinlich die wenigstens eine Landmarke in dem entsprechenden Voxel abgebildet ist.

In einem Verfahrensschritt eines Bestimmens DET-1 der Ausrichtung der wenigstens einen diagnostisch relevanten Schnittebene wird die Ausrichtung in Abhängigkeit der wenigstens einen Landmarke bestimmt.

Mit anderen Worten wird die Ausrichtung in Abhängigkeit der Position der wenigstens einen Landmarke bestimmt. Insbesondere kann die Ausrichtung in Abhängigkeit von Positionen von mehr als einer Landmarke bestimmt werden. Insbesondere kann die Ausrichtung in Abhängigkeit einer Auswahl der bestimmten Positionen der Landmarken bestimmt werden. Dabei kann die Auswahl für die diagnostisch relevante Schnittebene vordefiniert bzw. vorgegeben sein. Für verschiedene diagnostisch relevante Schnittebenen können verschiedene Auswahlen von Landmarken bzw. deren Positionen berücksichtigt werden. Mit anderen Worten kann die Ausrichtung verschiedener diagnostisch relevanter Schnittebenen von verschiedenen Landmarken abhängen.

Insbesondere beim Bestimmen DET-1 der Ausrichtung ein räumlicher Zusammenhang zwischen der Position der wenigstens einen Landmarke und der diagnostisch relevanten Schnittebene berücksichtigt werden. Insbesondere kann die wenigstens eine Landmarke in der diagnostisch relevanten Schnittebene liegen.

Insbesondere kann beim Bestimmen DET-1 der Ausrichtung der diagnostisch relevanten Schnittebene die Wahrscheinlichkeitsverteilung berücksichtigt werden. Wenn beispielsweise drei Landmarken in der diagnostisch relevanten Schnittebene liegen sollen, kann die Ausrichtung der diagnostisch relevanten Schnittebene derart bestimmt werden, dass das Mittel der Wahrscheinlichkeiten der Bereiche, durch die die diagnostisch relevante Schnittebene verläuft, für alle drei Landmarken maximal ist.

In Ausführungen der Erfindung kann beim Bestimmen DET-1 der Ausrichtung zusätzlich ein Vorwissen bezüglich der Ausrichtung berücksichtigt werden. Das Vorwissen kann beispielsweise eine bekannte Ausrichtung relativ zu einer Standardausrichtung in dem MRT System (coronal, sagittal, axial) berücksichtigen. Alternativ oder zusätzlich kann das Vorwissen eine Lage des Herzens o.ä. berücksichtigen.

Die diagnostisch relevante Schnittebene kann insbesondere eine der folgenden Schnittebenen sein: Vier-Kammern-Ebene, Drei-Kammern-Ebene, Zwei-Kammern-Ebene, Vertikale-Lange-Achse, Horizontale-Lange-Achse, Kurze-Achse.

Eine theoretische Lage der genannten Schnittebenen ist beispielsweise in Maier et al., "Kardiale Magnetresonanztomographie - Anatomie und Planung", Journal für Kardiologie - Austrian Journal of Cardiology 2003; 10: 3-7 beschrieben.

Die wenigstens eine diagnostisch relevante Schnittebene kann in Ausführungen der Erfindung eine der folgenden Schnittebenen sein:
- Vertikale-Lange-Achse (engl.: vertical long axis, Akronym: VLA)
- Horizontale-Lange-Achse (engl.: horizontal long axis, Akronym: HLA)
- Drei-Kammern-Ebene (engl.: three chamber view, Akronym: 3CH)
- Vier-Kammern-Ebene (engl.: four chamber view, Akronym: 4CH)
- Kurze Achse (engl.: short axis, Akronym: SAX)
- Rechtes Ventrikel Zwei-Kammern-Ebene (engl.: right ventricle two chamber view, Akronym: RV-2CH)
- Rechtes Ventrikel Drei-Kammern-Ebene (engl.: right ventricle three chamber view, Akronym: RV-3CH)
- Aortenfluss-Ebene (engl.: Aortic Flow Plane)
- Ebene des linksvetrikulären Ausflusstrakts (engl.: left ventricular outflow tract plane, Akronym: LVOT)
- Ebene der Mitralklappe (engl.: mitral valve plane, Akronym: MV Plane).

Dabei kann die Ausrichtung der Schnittebenen von den im Folgenden hinter den Schnittebenen in Klammern angegebenen Landmarken bzw. deren Positionen abgeleitet werden bzw. abhängen:
- VLA (LV apex, MV midpoint + Vorwissen: Parasagittal)
- HLA (LV apex, MV midpoint + Vorwissen: orthogonal zu VLA)
- 3CH (LV apex, MV midpoint, AV midpoint)
- 4CH (LV apex, MV midpoint, RV extent)
- SAX (Mittelpunkt von LV apex und MV midpoint, RV extent + Vorwissen: orthogonal zu VLA und HLA)
- RV-2CH (RV apex, TV midpoint + Vorwissen: Parasagittal)
- RV-3CH (RV apex, TV midpoint, PA center point)
- Aortic Flow Plane (Aortic bulb (anterior), Aortic bulb (posterior), Aortic bulb (left), Aortic bulb (right))
- LVOT (Aortic bulb (anterior), Aortic bulb (posterior) + Vorwissen: Schicht im Mittelpunkt der beiden Landmarken, orthogonal zum Verbindungsvektor der Landmarken)
- MV Plane (MV insertion (septal), MV insertion (lateral), MV insertion (superior), MV insertion (inferior)).

In einem Verfahrensschritt eines Bereitstellens PROV-2 der Ausrichtung der wenigstens einen diagnostisch relevanten Schnittebene, wird die Ausrichtung insbesondere mit einer Schnittstelle SYS.IF bereitgestellt. Insbesondere kann die Ausrichtung in Form eines Vektors bezüglich eins Koordinatensystems des MRT Systems bereitgestellt werden. Alternativ kann die Ausrichtung als Ausrichtung eines Magnetfeldgradienten bereitgestellt werden, welcher dazu ausgebildet ist, die Protonen in der diagnostisch relevanten Schnittebene derart auszurichten, dass sie mit einem Anregungspuls angeregt werden können.

**Figur 2** zeigt ein zweites Ausführungsbeispiel eines computerimplementierten Verfahrens zum Bestimmen einer Ausrichtung wenigstens einer diagnostisch relevanten Schnittebene für eine Herzbildgebung in einem dreidimensionalen Magnet-Resonanz-Tomographie Bilddatensatz.

Das bezüglich Figur 1 beschriebene erste Ausführungsbeispiel ist in dem hier beschriebenen zweiten Ausführungsbeispiel mit einem Verfahrensschritt eines Bereitstellens PROV-3 wenigstens eines ersten Aufnahmeparameterwertes weitergebildet. Die anderen Verfahrensschritte sind gemäß der Beschreibung zu Figur 1 ausgebildet.

Der wenigstens eine erste Aufnahmeparameterwert ist zum Steuern eines medizintechnischen MRT Systems 10 zum Erfassen eines zweidimensionalen Schnittbildes von der wenigstens einen diagnostisch relevanten Schnittebene ausgebildet.

Insbesondere ist der erste Aufnahmeparameterwert derart ausgebildet, dass eine Gradientensteuereinheit 19 eines MRT Systems 10 mittels des ersten Aufnahmeparameterwertes eine Gradientenspuleneinheit 18 des MRT Systems 10 ansteuern kann. Das MRT System 10 ist in der Beschreibung zu Figur 7 erläutert.

Insbesondere kann der erste Aufnahmeparameterwert derart ausgebildet sein, dass die Gradientenspuleneinheit 18 einen Magnetfeldgradienten erzeugt, der senkrecht auf die diagnostisch relevanten Schnittebene ausgerichtet ist. Mit anderen Worten kann der erste Aufnahmeparameterwert eine Richtung des Magnetfeldgradienten vorgeben. Der Magnetfeldgradient bildet dabei gemeinsam mit einem homogenen Grundmagnetfeld, welches von einem Grundmagneten 16 erzeugt wird, ein Magnetfeld, in welchem das Untersuchungsobjekt angeordnet ist.

Insbesondere wird die Gradientenspuleneinheit 18 mit dem ersten Aufnahmeparameterwert derart angesteuert, dass die von dem Untersuchungsobjekt umfassten Protonen-Spins in der diagnostisch relevanten Schnittebene in einer bestimmten Larmor-Frequenz präzedieren bzw. rotieren. Dabei ist die Larmor-Frequenz proportional zu einer Magnetfeldstärke des Magnetfeldes. Insbesondere kann der erste Aufnahmeparameterwert somit eine Stärke des Magnetfeldgradienten vorgeben. Insbesondere ist der Magnetfeldgradient innerhalb der diagnostisch relevanten Schnittebene konstant. Insbesondere präzedieren dann alles Spins in der diagnostisch relevanten Schnittebene mit derselben Larmor-Frequenz. Insbesondere können die Spins in der diagnostisch relevanten Schnittebene dann mit einem Anregungspuls genau dieser Frequenz angeregt werden. Das Signal, das die Spins beim Relaxieren in dem Magnetfeld aussenden, kann dann erfasst werden. Aus diesem Signal kann das zweidimensionale Schnittbild der diagnostisch relevanten Schnittebene rekonstruiert werden. Die angeregten Spins sind in einer Schicht in dem Untersuchungsobjekt senkrecht zu dem Magnetfeldgradienten angeordnet. Die Schicht ist insbesondere in der diagnostisch relevanten Schnittebene angeordnet.

In Ausführungen der Erfindung kann der erste Aufnahmeparameterwert eine "Steilheit" des Magnetfeldgradienten vorgeben. Durch die "Steilheit" des Magnetfeldgradienten kann die Dicke der Schicht der angeregten Spins senkrecht zu der diagnostisch relevanten Schnittebene vorgegeben werden. Je flacher der Magnetfeldgradient, desto mehr Spins präzedieren wenigstens näherungsweise mit der Frequenz des Anregungspulses und werden somit angeregt, desto dicker ist somit die Schicht. Je steiler der Magnetfeldgradient, desto weniger Spins präzedieren mit der Frequenz des Anregungspulses und desto dünner ist somit die angeregte Schicht.

Der wenigstens eine erste Aufnahmeparameterwert wird in dem Verfahrensschritt des Bereitstellens PROV-3 insbesondere von der Ausrichtung der diagnostisch relevanten Schnittebene abgeleitet. Insbesondere ist der erste Aufnahmeparameterwert derart ausgebildet, dass durch den ersten Aufnahmeparameterwert ein Magnetfeldgradient erzeugt werden kann, der Spins innerhalb der diagnostisch relevanten Schnittebene anregt. Mit anderen Worten ist der erzeugte Magnetfeldgradient dann senkrecht auf die diagnostisch relevante Schnittebene bzw. parallel zu einem Normalenvektor auf die diagnostisch relevante Schnittebene ausgerichtet.

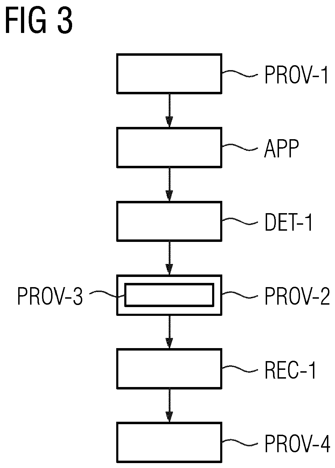

**Figur 3** zeigt ein drittes Ausführungsbeispiel eines computerimplementierten Verfahrens zum Bestimmen einer Ausrichtung wenigstens einer diagnostisch relevanten Schnittebene für eine Herzbildgebung in einem dreidimensionalen Magnet-Resonanz-Tomographie Bilddatensatz.

Das bezüglich Figur 2 beschriebene zweite Ausführungsbeispiel ist in dem hier beschriebenen dritten Ausführungsbeispiel mit einem Verfahrensschritt eines Erfassens REC-1 eines zweidimensionalen Schnittbildes und eines Bereitstellens PROV-4 des zweidimensionalen Schnittbildes weitergebildet. Die anderen Verfahrensschritte sind gemäß der Beschreibung zu den Figuren 1 und 2 ausgebildet.

In dem Verfahrensschritt des Erfassens REC-1 des zweidimensionalen Schnittbildes wird das zweidimensionale Schnittbild mit dem MRT System 10 in Abhängigkeit des ersten Aufnahmeparameterwertes erfasst. Das Grundprinzip des MRT Systems 10 ist in der Beschreibung zu Figur 7 erläutert.

Der Verfahrensschritt des Erfassens REC-1 kann insbesondere einen Verfahrensschritt eines Empfangens bzw. Auslesens eines Signals und einen Verfahrensschritt eines Rekonstruierens des zweidimensionalen Schnittbildes aus dem Signal umfassen.

Das Signal kann insbesondere beim Relaxieren der Spins in dem Magnetfeld erfasst empfangen werden. Dabei kann das Signal mit einer Antenne, insbesondere einer Spule, empfangen werden. Die Antenne kann eine System-Spule 20 oder eine Lokalspule 38 sein.

In dem Verfahrensschritt des Rekonstruierens wird das zweidimensionale Schnittbild basierend auf dem empfangenen Signal rekonstruiert. Insbesondere kann die Rekonstruktion auf einer gefilterten Rückpropagation (engl.: filtered backpropagation) basieren.

In dem Verfahrensschritt des Bereitstellens PROV-4 des zweidimensionalen Schnittbildes wird das Schnittbild insbesondere mit einer Schnittstelle SYS.IF bereitgestellt.

In Ausführungen der Erfindung kann das Schnittbild einem medizinischen Bedienpersonal bereitgestellt werden. Dafür kann das Schnittbild auf einer Anzeigeeinheit, insbesondere einem Monitor oder einem Bildschirm, angezeigt werden.

Alternativ oder zusätzlich kann das zweidimensionale Schnittbild auf einem Server, insbesondere in einer Datenbank, gespeichert werden. Dabei kann der Server ein lokaler Server oder ein Cloud-Server sein. Insbesondere kann die Datenbank ein PACS sein.

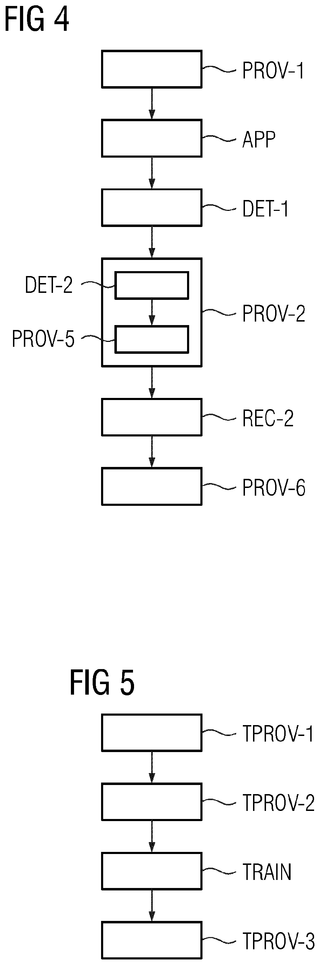

**Figur 4** zeigt ein viertes Ausführungsbeispiel eines computerimplementierten Verfahrens zum Bestimmen einer Ausrichtung wenigstens einer diagnostisch relevanten Schnittebene für eine Herzbildgebung in einem dreidimensionalen Magnet-Resonanz-Tomographie Bilddatensatz.

Das bezüglich Figur 1 beschriebene erste Ausführungsbeispiel ist in dem hier beschriebenen vierten Ausführungsbeispiel mit den Verfahrensschritten eines Bestimmens DET-2 einer Ausdehnung eines dreidimensionalen Volumenbildes senkrecht zu der diagnostisch relevanten Schnittebene, eines Bereitstellens PROV-5 wenigstens eines zweiten Aufnahmeparameterwertes, eines Erfassens REC-2 des dreidimensionalen Volumenbildes und eines Bereitstellens PROV-6 des dreidimensionalen Volumenbildes weitergebildet. Die anderen Verfahrensschritte sind gemäß der Beschreibung zu Figur 1 ausgebildet.

Das dreidimensionale Volumenbild ist eine dreidimensionale Darstellung bzw. Abbildung des Herzens bzw. des Teils des Herzens. In dem dreidimensionalen Volumenbild ist auch das Herz in der diagnostisch relevanten Schnittebene abgebildet. Die Größe des dreidimensionalen Volumenbildes wird dabei von der diagnostisch relevanten Schnittebene und einer Ausdehnung des dreidimensionalen Volumenbildes senkrecht zu der diagnostisch relevanten Schnittebene vorgegeben bzw. aufgebspannt. Die diagnostisch relevante Schnittebene kann dabei an einer beliebigen Position des dreidimensionalen Volumenbildes liegen. Insbesondere kann die diagnostisch relevante Schnittebene eine Seitenfläche des dreidimensionalen Volumenbildes bilden. Alternativ kann die diagnostisch relevante Schnittebene beispielsweise in der Mitte des dreidimensionalen Volumenbildes angeordnet sein.

In dem Verfahrensschritt des Bestimmens DET-2 der Ausdehnung des dreidimensionalen Volumenbildes wird dessen Ausdehnung senkrecht zu der diagnostisch relevanten Schnittebene bestimmt. Insbesondere kann die Ausdehnung derart bestimmt werden, dass eine Anatomie des Herzens in dem dreidimensionalen Volumenbild abgebildet ist. Mit anderen Worten kann die Ausdehnung in Abhängigkeit einer anatomischen Gegebenheit des Herzens bestimmt werden. Die Anatomie bzw. die anatomische Gegebenheit kann dabei in Abhängigkeit des dreidimensionalen Bilddatensatzes bestimmt werden. Die anatomische Gegebenheit kann dabei beispielsweise von einer oder mehreren Landmarken abhängen. Beispielsweise kann die Ausdehnung derart ausgebildet sein, dass ein Aortenbogen des Herzens in dem Volumenbild abgebildet ist. Die Ausdehnung kann insbesondere in einer metrischen Einheit angegeben werden. Beispielsweise kann die Ausdehnung 0,1cm oder 0,5cm oder 1cm oder 2cm umfassen.

In dem Verfahrensschritt des Bereitstellens PROV-5 wird der wenigstens eine zweite Aufnahmeparameterwert zum Steuern des MRT Systems beim Erfassen des dreidimensionalen Volumenbildes bereitgestellt. Der wenigstens eine zweite Aufnahmeparameterwert wird dabei von der Ausrichtung der diagnostisch relevanten Schnittebene und der Ausdehnung abgeleitet. Insbesondere ist der wenigstens eine zweite Aufnahmeparameterwert derart ausgebildet, dass mit ihm das MRT System derart angesteuert werden kann, dass das dritte Volumenbild erfasst werden kann. Der wenigstens eine zweite Aufnahmeparameterwert kann wenigstens teilweise wie bezüglich des ersten Aufnahmeparameterwertes in der Beschreibung zu Figur 2 beschrieben ausgebildet sein. Zusätzlich kann der wenigstens eine zweite Aufnahmeparameterwert eine Information darüber umfassen, wie eine Stärke des Magnetfeldgradienten variiert werden muss, damit bei konstantem Anregungspuls iterativ die Spins in dem gesamten von dem Volumenbild umfassten Bereich des Herzens angeregt werden und das entsprechende Signal erfasst werden kann. Mit anderen Worten kann der wenigstens eine zweite Aufnahmeparameterwert eine Abfolge von Stärken des Magnetfeldgradienten umfassen. Alternativ kann der wenigstens eine zweite Aufnahmeparameterwert eine Information über eine Veränderung der Frequenz des Anregungspulses umfassen, damit bei gleichbleibendem Magnetfeldgradienten durch die Änderung der Frequenz des Anregungspulses die Spins in dem gesamten Bereich des in dem Volumenbild abgebildeten Bereiches angeregt werden. Mit anderen Worten kann der wenigstens eine zweite Aufnahmeparameterwert eine Abfolge an Frequenzen des Anregungspulses umfassen.

In dem Verfahrensschritt des Erfassens REC-2 des dreidimensionalen Volumenbildes wird das dreidimensionale Volumenbild mit dem MRT System erfasst. Dabei wird das MRT System mit dem wenigstens einen zweiten Anregungspuls angesteuert. Das Erfassen REC-2 des dreidimensionalen Volumenbildes kann analog zu dem in der Beschreibung zu Figur 3 beschriebenen Erfassen REC-1 des zweidimensionalen Schnittbildes erfolgen. Im Unterschied dazu, wird während des Erfassens die Frequenz des Anregungspulses und/oder die Stärke des Magnetfeldgradienten variiert, um die Spins in dem gesamten, in dem Volumenbild abgebildeten Bereich anzuregen.

In dem Verfahrensschritt des Bereitstellens PROV-6 des dreidimensionalen Volumenbildes, wird das dreidimensionale Volumenbild insbesondere mittels einer Schnittstelle SYS.IF bereitgestellt. Insbesondere kann das dreidimensionale Volumenbild einem medizinischen Bedienpersonal angezeigt werden. Alternativ oder zusätzlich kann das dreidimensionale Volumenbild in einer Datenbank oder auf einem Server abgespeichert werden. Das Bereitstellen PROV-6 des dreidimensionalen Volumenbildes kann analog zu dem Bereitstellen PROV-4 des zweidimensionalen Schnittbildes gemäß der Beschreibung zu Figur 3 erfolgen.

**Figur 5** zeigt ein Ausführungsbeispiel eines computerimplementierten Verfahrens zum Bereitstellen einer trainierten Funktion zum Bestimmen einer Position wenigstens einer Landmarke in einem dreidimensionalen Magnet-Resonanz-Tomographie Bilddatensatz.

Der dreidimensionale Bilddatensatz und die wenigstens eine Landmarke sind, insbesondere wie oben bezüglich den Figuren 1 bis 4 beschrieben, ausgebildet.

Die trainierte Funktion ist zum Anwenden in dem Verfahrensschritt des Anwendens APP der trainierten Funktion gemäß der Beschreibung zu Figur 1 ausgebildet.

Mit anderen Worten umfassen die Eingangsdaten der trainierten Funktion die dreidimensionalen Bilddaten und die Ausgangsdaten die Position der wenigstens einen Landmarke.

Im Folgenden wird das Verfahren zum Trainieren der trainierten Funktion beschrieben.

In einem Verfahrensschritt eines Empfangens TPROV-1 von wenigstens einem dreidimensionalen Trainings-Bilddatensatz, wird der wenigstens einen dreidimensionale Trainings-Bilddatensatz mittels einer Trainings-Schnittstelle TSYS.IF empfangen. Dabei kann der dreidimensionale Trainings-Bilddatensatz analog zu dem dreidimensionalen Bilddatensatz gemäß der Beschreibung zu Figur 1 ausgebildet sein. Insbesondere kann der dreidimensionale Trainings-Bilddatensatz von einem Server, insbesondere von einer Datenbank auf dem Server empfangen werden. Der Server kann dabei ein lokaler Server oder ein Clou-Sever sein. Die Datenbank kann beispielsweise ein PACS sein.

Insbesondere kann eine Mehrzahl von dreidimensionalen Trainings-Bilddatensätzen empfangen werden.

In einem Verfahrensschritt eines Empfangens TPROV-2 von wenigstens einem annotierten dreidimensionalen Trainings-Bilddatensatz, wird der wenigstens eine annotierte dreidimensionale Trainings-Bilddatensatz mit der Trainings-Schnittstelle TSYS.IF empfangen. Der annotierte dreidimensionale Trainings-Bilddatensatz kann von dem Server, insbesondere von der Datenbank auf dem Server, empfangen werden.

Wenn eine Mehrzahl von dreidimensionalen Trainings-Bilddatensätzen in dem Verfahrensschritt des Empfangens TPROV-1 wenigstens eines dreidimensionalen Trainings-Bilddatensatzes empfangen wird, wird für jeden der dreidimensionalen Trainings-Bilddatensätzen wenigstens ein annotierter dreidimensionaler Trainings-Bilddatensatz empfangen.

Der annotierte dreidimensionale Trainings-Bilddatensatz basiert auf dem dreidimensionalen Trainings-Bilddatensatz. Insbesondere ist in dem annotierten dreidimensionalen Trainings-Bilddatensatz die Position der wenigstens einen Landmarke annotiert bzw. markiert bzw. eingezeichnet bzw. gekennzeichnet. Mit anderen Worten umfasst der annotierte dreidimensionale Trainings-Bilddatensatz die Position der wenigstens einen Landmarke in dem dreidimensionalen Trainings-Bilddatensatz.

Insbesondere kann die Position der wenigstens einen Landmarke den Voxel in dem dreidimensionalen Trainings-Bilddatensatz angeben, der die wenigstens eine Landmarke abbildet. Insbesondere kann dann der annotierte dreidimensionale Trainings-Bilddatensatz eine Koordinate des Voxels in der Voxelmatrix umfassen.

Alternativ kann die Position der wenigstens einen Landmarke einen oder mehrere Bereiche in dem dreidimensionalen Trainings-Bilddatensatz angeben. Dabei kann jedem Bereich eine Wahrscheinlichkeit zugeordnet sein, mit der die wenigstens eine Landmarke innerhalb des entsprechenden Bereiches abgebildet ist. Insbesondere kann dann der annotierte dreidimensionale Trainings-Bilddatensatz einer Voxelmatrix gemäß der Voxelmatrix des dreidimensionalen Trainings-Bilddatensatzes entsprechen, die in ein oder mehrere Bereiche unterteilt ist.

Insbesondere kann die Position von mehr als eine Landmarke in dem annotierten dreidimensionalen Trainings-Bilddatensatz annotiert sein. Alternativ kann für einen dreidimensionalen Trainings-Bilddatensatz für jede Landmarke ein annotierter dreidimensionaler Trainings-Bilddatensatz empfangen werden. Dabei kann in jedem annotierten Trainings-Bilddatensatz die Position einer anderen Landmarke annotiert sein.

Insbesondere kann der annotierte dreidimensionale Trainings-Bilddatensatz manuell annotiert sein. Mit anderen Worten kann die Position der wenigstens einen Landmarke von einem medizinischen Bedienpersonal manuell eingezeichnet bzw. annotiert bzw. markiert werden. Insbesondere kann das medizinische Bedienpersonal den Voxel markieren, der die wenigstens eine Landmarke abbildet. Alternativ kann das medizinische Bedienpersonal den oder die oben beschriebenen Bereiche als Position der wenigstens einen Landmarke einzeichnen.

In einem Verfahrensschritt eines Trainierens einer Funktion, wird die Funktion in Abhängigkeit des dreidimensionalen Trainings-Bilddatensatzes und in Abhängigkeit des annotierten dreidimensionalen Trainings-Bilddatensatzes trainiert.

Dafür wird die Funktion auf den wenigstens einen dreidimensionalen Trainings-Bilddatensatz angewendet. Dabei wird eine Position der wenigstens einen Landmarke in dem dreidimensionalen Trainings-Bilddatensatz bestimmt. Die derart bestimmte Position wird mit der Position in dem annotierten dreidimensionalen Trainings-Bilddatensatz verglichen. Wenn die beiden Positionen voneinander abweichen, wird die Funktion derart angepasst bzw. modifiziert, dass bei einem erneuten Anwenden der Funktion auf den dreidimensionalen Trainings-Bilddatensatz die bestimmte Position besser mit der Position gemäß dem annotierten dreidimensionalen Trainings-Bilddatensatz übereinstimmt. Dieses Anpassen beschreibt das Training der Funktion.

Insbesondere kann dieses Verfahren für die Mehrzahl von dreidimensionalen Trainings-Bilddatensätzen und die entsprechende Mehrzahl von annotierten dreidimensionalen Trainings-Bilddatensätzen wiederholt werden.

Insbesondere kann die Funktion iterativ weiter angepasst werden, bis ein Abbruchkriterium erreicht ist. Das Abbruchkriterium kann beispielsweise eine maximale Zahl an Iterationen und/oder eine maximale Zahl an dreidimensionalen Trainings-Bilddatensätzen und/oder ein Unterschreiten einer maximalen Abweichung der bestimmten Position und der Position gemäß dem annotierten dreidimensionalen Trainings-Bilddatensatz sein.

Die Funktion kann für die verschiedenen Landmarken getrennt trainiert werden. Alternativ kann die Funktion für alle Landmarken auf einmal trainiert werden. Insbesondere kann die Funktion für alle Landmarken auf einmal trainiert werden, wenn die Landmarken alle in einem annotierten dreidimensionalen Trainings-Bilddatensatz annotiert sind.

In einem Verfahrensschritt eines Bereitstellens TPROV-3 der trainierten Funktion, wird die trainierte Funktion, insbesondere mittels der Trainings-Schnittstelle TSYS-IF bereitgestellt. Insbesondere wird die trainierte Funktion für eine Anwendung in dem Verfahren gemäß einer der Figuren 1 bis 3 bereitgestellt. Insbesondere kann die trainierte Funktion für eine Verwendung auf einer Recheneinheit bereitgestellt werden. Insbesondere kann die trainierte Funktion in dem Verfahrensschritt des Bereitstellens TPROV-3 der trainierten Funktion auf einer Speichereinheit hinterlegt bzw. gespeichert werden.

**Figur 6** zeigt ein Bestimmungssystem SYS zum Bestimmen einer Ausrichtung wenigstens einer diagnostisch relevanten Schnittebene für eine Herzbildgebung in einem dreidimensionalen Magnet-Resonanz-Tomographie Bilddatensatz, **Figur 7** zeigt ein Trainingssystem TSYS zum Bereitstellen einer trainierten Funktion zum Bestimmen einer Position wenigstens einer Landmarke in einem dreidimensionalen Magnet-Resonanz-Tomographie Bilddatensatz.

Das dargestellte Bestimmungssystem SYS zum Bestimmen einer Ausrichtung wenigstens einer diagnostisch relevanten Schnittebene für eine Herzbildgebung in einem dreidimensionalen MRT Bilddatensatz ist dazu ausgebildet ein erfindungsgemäßes Verfahren zum Bestimmen einer Ausrichtung wenigstens einer diagnostisch relevanten Schnittebene für eine Herzbildgebung in einem dreidimensionalen MRT Bilddatensatz auszuführen. Das dargestellte Trainingssystem TSYS ist dazu ausgebildet ein erfindungsgemäßes Verfahren zum Bereitstellen der trainierten Funktion zum Bestimmen einer Position wenigstens einer Landmarke in einem dreidimensionalen Magnet-Resonanz-Tomographie Bilddatensatz auszuführen. Das Bestimmungssystem SYS umfasst eine Schnittstelle SYS.IF, eine Recheneinheit SYS.CU und eine Speichereinheit SYS.MU. Das Trainingssystem TSYS umfasst eine Trainings-Schnittstelle TSYS.IF, eine Trainings-Recheneinheit TSYS.CU und eine Trainings-Speichereinheit TSYS.MU.

Das Bestimmungssystem SYS und/oder das Trainingssystem TSYS kann insbesondere ein Computer, ein Mikrocontroller oder ein integrierter Schaltkreis (integrated circuit, IC) sein. Alternativ kann das Bestimmungssystem SYS und/oder das Trainingssystem TSYS ein reales oder virtuelles Computer-Netzwerk sein (eine technische Bezeichnung für ein reales Computer-Netzwerk ist "Cluster", eine technische Bezeichnung für ein virtuelles Computer-Netzwerk ist "Cloud"). Das Bestimmungssystem SYS und/oder das Trainingssystem TSYS kann als virtuelles System ausgebildet sein, welches auf einem Computer oder einem realen Computer-Netzwerk oder einem virtuellen Computer-Netzwerk ausgeführt wird (eine technische Bezeichnung ist "Virtualization").

Die Schnittstelle SYS.IF und/oder die Trainings-Schnittstelle TSYS.IF kann eine Hardware- oder Software-Schnittstelle sein (beispielsweise ein PCI bus, USB oder Firewire). Die Recheneinheit SYS.CU und/oder die Trainings-Recheneinheit TSYS.CU kann Hardware und/oder Software Bestandteile umfassen, beispielsweise einen Mikroprozessor oder einen sogenannten FPGA (Field Programmable Gate Way). Die Speichereinheit SYS.MU und/oder die Trainings-Speichereinheit TSYS.MU kann als nicht permanent arbeitender Arbeitsspeicher (Random Access Memory, RAM) oder als permanenter Massenspeicher (Festplatte, USB-Stick, SD-Karte, Solid State Disk (SSD)) ausgebildet sein.

Die Schnittstelle SYS.IF und/oder die Trainings-Schnittstelle TSYS.IF kann insbesondere eine Mehrzahl an Sub-Schnittstellen umfassen, die unterschiedliche Verfahrensschritte des jeweiligen erfindungsgemäßen Verfahrens ausführen. Mit anderen Worten kann die Schnittstelle SYS.IF und/oder die Trainings-Schnittstelle TSYS.IF als eine Mehrzahl an Schnittstellen SYS.IF und/oder Trainings-Schnittstellen TSYS.IF ausgebildet sein. Die Recheneinheit SYS.CU und/oder die Trainings-Recheneinheit TSYS.CU kann insbesondere eine Mehrzahl an Sub-Recheneinheiten umfassen, die unterschiedliche Verfahrensschritte des jeweiligen erfindungsgemäßen Verfahrens ausführen. Mit anderen Worten kann die Recheneinheit SYS.CU und/ oder die Trainings-Recheneinheit TSYS.CU als eine Mehrzahl an Recheneinheiten SYS.CU und/oder Trainings-Recheneinheiten TSYS.CU ausgebildet sein.

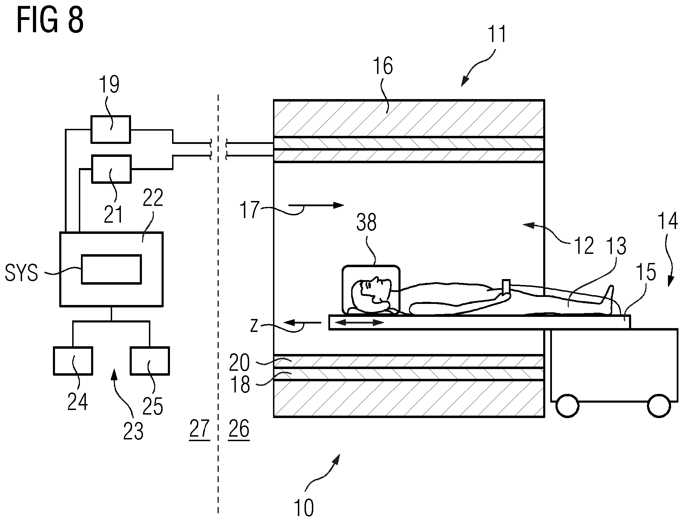

**Figur 8** zeigt ein Magnet-Resonanz-Tomographie System 10.

Das MRT System 10 ist schematisch darstellt. Das MRT System 10 umfasst eine von einer Magneteinheit gebildeten Scannereinheit 11. Zudem weist das MRT System 10 einen Aufnahmebereich 12 auf zu einer Aufnahme eines Untersuchungsobjektes 13, insbesondere eines Patienten. Der Aufnahmebereich 12 im vorliegenden Ausführungsbeispiel ist zylinderförmig ausgebildet und in einer Umfangsrichtung von der Scannereinheit 11, insbesondere von der Magneteinheit, zylinderförmig umgeben. Grundsätzlich ist jedoch eine davon abweichende Ausbildung des Aufnahmebereichs 12 jederzeit denkbar. Das Untersuchungsobjekt 13 kann mittels einer Lagerungsvorrichtung 14 des MRT Systems 10 in den Aufnahmebereich 12 geschoben und/oder gefahren werden. Die Lagerungsvorrichtung 14 weist hierzu eine innerhalb des Aufnahmebereichs 12 bewegbar ausgestaltete Liege 15 auf. Insbesondere ist hierbei die Liege 15 in Richtung einer Längserstreckung des Aufnahmebereichs 12 und/oder in z-Richtung bewegbar gelagert.

Die Scannereinheit 11, insbesondere die Magneteinheit, umfasst einen supraleitenden Grundmagneten 16 zu einem Erzeugen eines starken und insbesondere konstanten Grundmagnetfelds 17. Weiterhin weist die Scannereinheit 11, insbesondere die Magneteinheit, eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 des MRT Systems 10 gesteuert. Insbesondere kann eine Ausrichtung des Magnetfeldgradienten mit der Gradientensteuereinheit 19 gesteuert werden. Insbesondere kann die Gradientensteuereinheit 19 die Ausrichtung des Magnetfeldgradienten mit einem ersten Aufnahmeparameterwert steuern. Die Scannereinheit 11, insbesondere die Magneteinheit, umfasst weiterhin eine Hochfrequenz-Antennen-Einheit bzw. eine System-Spule 20 zu einer Anregung einer Polarisation, die sich in dem von dem Grundmagneten 16 erzeugten Grundmagnetfeld 17 einstellt. Die System-Spule 20 ist dabei fest innerhalb der Scannereinheit 11, insbesondere der Magneteinheit, angeordnet. Die System-Spule 20 wird von einer System-Spulen-Steuereinheit 21 des MRT Systems 10 gesteuert und strahlt hochfrequente Magnetresonanzsequenzen in den Aufnahmebereich 12 der Magnetresonanzvorrichtung 10 ein.

Für ein Erfassen von Magnetresonanzsignalen bzw. Signalen weist das MRT System 10 wenigstens eine lokale Hochfrequenzspule bzw. Lokalspule 38 auf, die um einen zu untersuchenden Bereich des Untersuchungsobjektes 13 positioniert werden kann. Eine Auswahl einer Lokalspule 38 für die anstehende Magnetresonanzuntersuchung erfolgt dabei in Abhängigkeit von dem zu untersuchenden Bereich des Untersuchungsobjektes 13. Beispielsweise eine lokale Kopf-Hochfrequenzspule für eine Kopfuntersuchung oder eine lokale Knie-Hochfrequenzspule für eine Knieuntersuchung etc. Die Lokalspule 38 umfasst dabei wenigstens ein oben beschriebenes Spulenelement. Insbesondere können, wenn die Lokalspule 38 mehr als ein Spulenelement umfasst, die Spulenelemente der Lokalspule 38 einzeln ausgelesen bzw. angesteuert werden.

Zu einer Steuerung des Grundmagneten 16, der Gradientensteuereinheit 19 und zur Steuerung der System-Spulen-Steuereinheit 21 weist das MRT System 10 eine Systemsteuereinheit 22 auf. Die Systemsteuereinheit 22 steuert zentral das MRT System 10, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von MRT Aufnahmen bzw. medizinischen Bilddaten, die während der Magnetresonanzuntersuchung erfasst werden.

Des Weiteren umfasst das MRT System 10 eine Benutzerschnittstelle 23, die mit der Systemsteuereinheit 22 verbunden ist. Steuerinformationen wie beispielsweise Bildgebungsparameter, sowie rekonstruierte MRT Aufnahmen können auf einer Anzeigeeinheit 24, beispielsweise auf zumindest einem Monitor, der Benutzerschnittstelle 23 für einen Nutzer bzw. ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem Nutzer eingegeben werden können.

Die Scannereinheit 11 des MRT Systems 10 ist zusammen mit der Lagerungsvorrichtung 14 innerhalb eines Untersuchungsraums 26 angeordnet. Die Systemsteuereinheit 22 dagegen ist zusammen mit der Benutzerschnittstelle 23 innerhalb eines Kontrollraums 27 angeordnet. Der Kontrollraum 27 ist getrennt von dem Untersuchungsraum 26 ausgebildet. Insbesondere ist der Untersuchungsraum 26 hinsichtlich einer Hochfrequenzstrahlung von dem Kontrollraum 27 abgeschirmt. Während einer Magnetresonanzuntersuchung bzw. einem Erfassen einer MRT Aufnahme befindet sich das Untersuchungsobjekt 13 innerhalb des Untersuchungsraums 26, dagegen befindet sich der Nutzer innerhalb des Kontrollraums 27.

Das dargestellte MRT System 10 kann selbstverständlich weitere Komponenten umfassen, die MRT Systeme 10 gewöhnlich aufweisen. Eine allgemeine Funktionsweise des MRT Systems 10 ist zudem dem Fachmann bekannt, so dass auf eine detaillierte Beschreibung der weiteren Komponenten verzichtet wird.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Computerimplementiertes Verfahren zum Bestimmen einer Ausrichtung wenigstens einer diagnostisch relevanten Schnittebene für eine Herzbildgebung in einem dreidimensionalen Magnet-Resonanz-Tomographie Bilddatensatz,
umfassend folgende Verfahrensschritte:
- Bereitstellen (PROV-1) des dreidimensionalen Bilddatensatzes,
- Anwenden (APP) einer trainierten Funktion auf den dreidimensionalen Bilddatensatz,
wobei eine Position wenigstens einer Landmarke bestimmt wird,
- Bestimmen (DET-1) der Ausrichtung der wenigstens einen diagnostisch relevanten Schnittebene in Abhängigkeit der wenigstens einen Landmarke,
- Bereitstellen (PROV-2) der Ausrichtung der wenigstens einen diagnostisch relevanten Schnittebene.

2. Verfahren nach Anspruch 1,
wobei der dreidimensionale Bilddatensatz wenigstens einen Teil eines Herzens abbildet,
wobei der dreidimensionale Bilddatensatz ein Übersichtsscan des Teils des Herzens ist.

3. Verfahren nach Anspruch 2,
wobei die wenigstens eine Landmarke eine der folgenden Landmarken ist:
Apex, Mitralklappe, Aortenklappe, Pulmonalklappe, Trikuspidalklappe.

4. Verfahren nach einem der Ansprüche 2 oder 3,
wobei die wenigstens eine diagnostisch relevante Schnittebene eine der folgenden Schnittebenen ist:
Vier-Kammern-Ebene, Drei-Kammern-Ebene, Zwei-Kammern-Ebene, Vertikale-Lange-Achse, Horizontale-Lange-Achse, Kurze-Achse.

5. Verfahren nach einem der vorhergehenden Ansprüche,
wobei in dem Verfahrensschritt des Anwendens der trainierten Funktion die Position der wenigstens einen Landmarke in Form einer Wahrscheinlichkeitsverteilung für die Position der wenigstens einen Landmarke in dem dreidimensionalen Bilddatensatz bestimmt wird.

6. Verfahren nach einem der vorhergehenden Ansprüche,
wobei der Verfahrensschritt des Bereitstellens (PROV-2) der Ausrichtung der wenigstens einen diagnostisch relevanten Schnittebene folgenden Verfahrensschritt umfasst:
- Bereitstellen (PROV-3) wenigstens eines ersten Aufnahmeparameterwertes zum Steuern eines medizintechnischen Magnet-Resonanz-Tomographie Systems zum Erfassen eines zweidimensionalen Schnittbildes von der wenigstens einen diagnostisch relevanten Schnittebene.

7. Verfahren nach Anspruch 6,
wobei der wenigstens eine erste Aufnahmeparameterwert von der Ausrichtung der wenigstens einen diagnostisch relevanten Schnittebene abgeleitet wird.

8. Verfahren nach einem der Ansprüche 6 oder 7,
wobei das Verfahren außerdem folgende Verfahrensschritte umfasst:
- Erfassen (REC-1) des zweidimensionalen Schnittbildes mit dem Magnet-Resonanz-Tomographie System in Abhängigkeit des wenigstens einen ersten Aufnahmeparameterwertes,
- Bereitstellen (PROV-4) des zweidimensionalen Schnittbildes.

9. Verfahren nach einem der Ansprüche 1 bis 5,
wobei das Verfahren außerdem folgende Verfahrensschritte umfasst:
- Bestimmen (DET-2) einer Ausdehnung eines dreidimensionalen Volumenbildes senkrecht zu der diagnostisch relevanten Schnittebene,
wobei das dreidimensionale Volumenbild von der diagnostisch relevanten Schnittebene und der Ausdehnung aufgespannt wird,
- Bereitstellen (PROV-5) wenigstens eines zweiten Aufnahmeparameterwertes zum Steuern eines Magnet-Resonanz-Tomographie Systems zum Erfassen des dreidimensionalen Volumenbildes, wobei der wenigstens eine zweite Aufnahmeparameterwert von der Ausrichtung der wenigstens einen diagnostisch relevanten Schnittebene und der Ausdehnung abgeleitet wird,
- Erfassen (REC-2) des dreidimensionalen Volumenbildes mit dem Magnet-Resonanz-Tomographie System in Abhängigkeit des wenigstens einen zweiten Aufnahmeparameterwertes,
- Bereitstellen (PROV-6) des dreidimensionalen Volumenbildes.

10. Verfahren nach einem der vorhergehenden Ansprüche,
wobei die trainierte Funktion auf einem neuronalen Faltungsnetzwerk und/oder einem U-Netzwerk basiert.

11. Computerimplementiertes Verfahren zum Bereitstellen einer trainierten Funktion zum Bestimmen einer Position wenigstens einer Landmarke in einem dreidimensionalen Magnet-Resonanz-Tomographie Bilddatensatz,
umfassend folgende Verfahrensschritte:
- Empfangen (TPROV-1) von wenigstens einem dreidimensionalen Trainings-Bilddatensatz,
- Empfangen (TPROV-2) von wenigstens einem annotierten dreidimensionalen Trainings-Bilddatensatz,
wobei der annotierte dreidimensionale Trainings-Bilddatensatz auf dem dreidimensionalen Trainings-Bilddatensatz basiert,
wobei in dem annotierten dreidimensionalen Trainings-Bilddatensatz die Position der wenigstens einen Landmarke annotiert ist,
- Trainieren (TRAIN) einer Funktion in Abhängigkeit des dreidimensionalen Trainings-Bilddatensatzes und des annotierten dreidimensionalen Trainings-Bilddatensatzes,
- Bereitstellen (TPROV-3) der trainierten Funktion.

12. Verfahren nach Anspruch 11,
wobei der annotierte dreidimensionale Trainings-Bilddatensatz durch manuelles Annotieren des dreidimensionalen Trainings-Bilddatensatzes erstellt wird.

13. Bestimmungssystem (SYS) zum Bestimmen einer Ausrichtung wenigstens einer diagnostisch relevanten Schnittebene für eine Herzbildgebung in einem dreidimensionalen Magnet-Resonanz-Tomographie Bilddatensatz,
umfassend eine Schnittstelle (SYS.IF) und eine Recheneinheit (SYS.CU),
wobei die Schnittstelle (SYS.IF) zum Bereitstellen (PROV-1) des dreidimensionalen Bilddatensatzes ausgebildet ist,
wobei die Recheneinheit (SYS.CU) zum Anwenden (APP) einer trainierten Funktion auf den dreidimensionalen Bilddatensatz ausgebildet ist,
wobei eine Position wenigstens einer Landmarke bestimmt wird, wobei die Recheneinheit (SYS.CU) außerdem zum Bestimmen (DET) der Ausrichtung der wenigstens einen diagnostisch relevanten Schnittebene in Abhängigkeit der wenigstens einen Landmarke ausgebildet ist,
wobei die Schnittstelle (SYS.IF) außerdem zum Bereitstellen (PROV-2) der Ausrichtung der wenigstens einen diagnostisch relevanten Schnittebene ausgebildet ist.

14. Magnet-Resonanz-Tomographie System (10) umfassend ein Bestimmungssystem (SYS) nach Anspruch 13,
wobei das Magnet-Resonanz-Tomographie System (10) zum Erfassen (REC-1, REC-2) des dreidimensionalen Bilddatensatzes und/oder eines zweidimensionalen Schnittbildes ausgebildet ist.

15. Computerprogrammprodukt mit einem Computerprogramm, welches direkt in einen Speicher (SYS.MU) eines Bestimmungssystems (SYS) ladbar ist, mit Programmabschnitten, um alle Schritte des Verfahrens nach einem der Ansprüche 1 bis 10 auszuführen, wenn die Programmabschnitte von dem Bestimmungssystem (SYS) ausgeführt werden

16. Computerlesbares Speichermedium, auf welchem von einem Bestimmungssystem (SYS) lesbare und ausführbare Programmabschnitte gespeichert sind, um alle Schritte des Verfahrens nach einem der Ansprüche 1 bis 10 auszuführen, wenn die Programmabschnitte von dem Bestimmungssystem (SYS) ausgeführt werden.
